(19) **Europäisches Patentamt / European Patent Office / Office européen des brevets**

(11) **EP 1 770 545 A2**

(12) # EUROPEAN PATENT APPLICATION

(43) Date of publication:
04.04.2007 Bulletin 2007/14

(51) Int Cl.:
*G06F 17/00* $^{(2006.01)}$

(21) Application number: **06124991.8**

(22) Date of filing: **02.07.2003**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br><br>(30) Priority: **30.10.2002 US 422111 P**<br><br>(62) Document number(s) of the earlier application(s) in accordance with Art. 76 EPC:<br>**03809894.3 / 1 555 935**<br><br>(71) Applicant: **DPCOM AS**<br>**0349 Oslo (NO)** | (72) Inventor: **Eide, Per Kristian**<br>**0779 OSLO (NO)**<br><br>(74) Representative: **Johansson Webjörn, Ingmari**<br>**Zacco Sweden AB,**<br>**Box 23101**<br>**104 35 Stockholm (SE)**<br><br>Remarks:<br>This application was filed on 29 - 11 - 2006 as a divisional application to the application mentioned under INID code 62. |

(54) **Method for analysis of single pulse pressure waves**

(57) This invention relates to a method for analysing pressure-signals derivable from pressure measurements on or in a body of a human being or animal, comprising the steps of identifying during given time sequences in a series of time sequences the single pressure waves, including related parameters [pressure amplitude ΔP, latency (ΔT), rise time coefficient (ΔP/ΔT)], determining numbers of single pressure waves with pre-selected combinations of two or more of said single pressure wave parameters during said time sequence. For the time sequences is further determined the balanced positions of single wave parameters. Two dimensional values of balanced position may be presented as a one dimensional value after weighting of the matrix cells. The signal processing method may be used for more optimal detection of single pressure waves by means of non-invasive sensor devices.

EP 1 770 545 A2

**Description**

**BACKGROUND OF THE INVENTION**

**Field of the Invention**

Monitoring of pressures within human body cavities has an important role in diagnosis and management of a large number of diseases and clinical conditions. The present invention relates to a method for analyzing pressure signals derivable from pressure measurements on or in a body of a human being or animal, comprising the steps of sampling said signals at specific intervals, and converting the pressure signals into pressure-related digital data with a time reference.

More specifically, the invention relates to methods as defined in the preamble of attached independent claims 1 and 94.

**Related Art**

Continuous monitoring of pressures in humans and animals has a widespread place. During continuous pressure monitoring, today's existing technology, not the inventive technology (hereafter referred to as conventional or current technology) calculates a mean or area under curve of several seconds of pressure recordings. For example, for a given time sequence of 6 seconds, mean pressure may be computed as the sum of all pressure sample levels divided by the numbers of samples. Most modem monitors update the calculated pressure value each 5-10 seconds. Thereby information within the single waves is lost. Whether or not the mean pressure corresponds to single pressure waves during said time sequence is not known. Therefore, absolute numbers of systolic, mean and diastolic pressures shown on the scope of vital signs monitors do not reveal single wave distribution. The basis for this praxis is the assumption of a linear relationship between mean pressure and amplitude of the single waves.

There are several problems with the current strategies of assessing continuous pressure recordings. Current technology uses calibration of pressures against a zero pressure level, usually the atmospheric pressure. This situation raises various problems, such as drift of zero pressure level during a period of recording. Differences in absolute zero pressure levels may cause false or inaccurate differences in pressures between different pressure recordings, making it difficult to compare pressure curves. Other causes of erroneous continuous pressure recordings are sensor failure, misplacement of pressure sensor, low quality of sensor signals related to movement of patient, and low signal-noise ratio of other reasons. Whether the quality of pressure signals is good or bad may be difficult to decide according to current strategies of assessing continuous pressure signals. The present invention aims at solving these problems, introducing a new strategy of analysis of pressure related digital data, including assessment of the single pressure waves.

A continuous pressure signal fluctuates over time related to the cardiac beats. In the human or animal body cavities, single pressure waves are built up from the waves created by each of the cardiac pulsation's. For example, the intracranial and arterial blood pressure waves are intimately related as the intracranial pressure waves arise from the contractions of the left cardiac ventricle. Each heart beat results in a pulse pressure wave, termed single pressure wave. Related to the cardiac beats, these waves have a diastolic minimum pressure and a subsequent systolic maximum pressure. When it has not previously been possible to take the knowledge of single wave parameters into daily clinical practice, this situation is related to the facts that heart rate is variable, single waves fluctuate a lot over time, and the inter-individual variation is large. So-called spectrum analysis or Fourier analysis assesses fluctuations in pressure, but not by analyzing the single pressure waves.

Non-invasive pressure monitoring is partially established for blood pressure and ocular pressure monitoring, though no methods or devices allows for continuous single wave monitoring with identification of single wave distribution. In particular, applanation tonometry is a non-invasive method for intraocular pressure measurement, blood pressure measurement, and measurements of intracranial pressure in infants.

**SUMMARY OF THE INVENTION**

There are a number of human or animal body cavities in which pressures may be recorded for diagnostic and therapeutic reasons. For example, pressures in a human or animal body cavity relate to arterial blood pressure, intracranial pressure, cerebrospinal fluid pressure, ocular pressure, urinary tract pressure, gastrointestinal tract pressure. The present invention primarily was designed for analysis of pressure signals derivable from monitoring of single waves in blood vessels, the intracranial compartment, the cerebrospinal fluid (CSF) system, the ocular bulb and the urinary tract and bladder. These cavities represent, however, no limitation in the context of the invention. Other cavities may be the esophageal tract, the anal canal, and others not specified. Thus, this invention is not limited to analysis of pressures from only some particular human or animal body cavities, as the invention relates to a generic method for analysis of pressure derivable signals.

This invention relates to analysis of continuous pressure related signals. Such pressure related data may be derived from a variety of pressure sensors and pressure transducers. Independent of the type of sensor, a continuous pressure signal is measured, providing the opportunity for sampling of single waves. Examples of such sensors are solid or fiber-optic mechanical sensors for invasive monitoring, and sensors for invasive monitoring of pressure within a fluid system such as arterial or venous blood vessels, cerebrospinal fluid, or urinary bladder/tract. There are various other types of sensors providing signals indicative of pressures. Examples are sensors for non-invasive measurement of blood pressure, using principles of Doppler technology, or measurement of oxygen saturation, and non-invasive measurements of intracranial pressure using Doppler technology or acoustic signals. The most well-known principle of non-invasive pressure monitoring uses the principles of applanation tonometry. For example, applanation tonometry is used for monitoring of fontanel pressure in infants, and ocular pressure and arterial blood pressure. The unique with the present invention is the opportunity for determining single wave distribution by more optimal detection of single pressure waves using the inventive method of analyzing single pressure waves.

More specifically and in a first aspect of the invention, the method for a selected time sequence comprises the further inventive steps of:

> a) identifying from said digital data the single pressure waves in said pressure signals,
> b) computing absolute mean pressure for said single pressure waves,
> c) computing single pressure wave related parameters of said single pressure waves,
> d) identifying numbers of single pressure waves with pre-selected parameter values of such waves with respect to amplitude, latency and rise time coefficient,
> e) plotting the numbers of occurrences of single pressure waves with pre-selected values of amplitude and latency in a first matrix,
> f) plotting the numbers of occurrences of single pressure waves with pre-selected values of rise time coefficients in a second matrix,
> g) determining balanced position of amplitude and latency combinations in said first matrix
> h) determining balanced position of rise time coefficients in said second matrix, and
> i) presenting the balanced positions obtained in steps g) and/ or h) as numerical values or as related to weighted values.

Further embodiments of this first aspect of the invention are defined in sub-claims 2 - 93.

In a second aspect of the invention, the method is applied for more optimum single pressure wave detection, wherein the method for analysis comprises the further steps of:

- identifying said single pressure wave related parameters during short time sequences, e.g. 3 seconds of duration,
- establishing an analysis output based on said determining steps for single pressure wave related parameters during said time sequence,
- establishing a deliverable first control signal related to said single pressure wave analysis output for each of said time sequences, wherein
- said first control signal is determined according to one or more selectable criteria for said analysis output,

wherein the method comprises the further steps of:

- modifying said deliverable first control signal into a regulator deliverable second control signal, said second control signal corresponding to said first deliverable control signal,
- delivering said second control signal to a sensor-regulating device, causing modifications of performance of said sensor-regulating device.

Further embodiments of this second aspect of the invention are defined in sub-claims 95 - 109.

Thus, this invention is not limited to specific types of pressure signals, however, the signal has to be continuous for a given time sequence. During continuous pressure monitoring, single pressure waves are sampled along with a time reference. Analog pressure signals are converted into digital pressure-related data. Since each wave represents a heart beat, the heart rate is known when the sampling rate is known. Determination of single wave distribution may be used in the real-time and online monitoring of pressures. Processing of single waves may be performed after sampling of pressure signals. Though data processing is performed with some delay, monitoring is real-time since the delay has no significance for the observed phenomenon. During identification of the single pressure waves, the continuous pressure signals undergo filtering and concatenation procedures wherein noise signals are removed. Each single pressure wave is identified according to the diastolic minimum ($P_{min}$) and systolic maximum ($P_{max}$) values. False $P_{min}$ and $P_{max}$ values

are removed. Identification of correct $P_{min}/P_{max}$ values are made by means of pre-determined thresholds for the single wave amplitude ($\Delta P$), latency ($\Delta T$) and rise time coefficient ($\Delta P/\Delta T$) values. For the correctly identified single pressure waves, the single wave parameters [amplitude ($\Delta P$), latency ($\Delta P$), and rise time coefficient ($\Delta P/\Delta T$)] are determined for short time sequences (e.g. each 5 seconds). Such short time sequences with identified single pressure waves are accepted or rejected according to selected criteria. The latencies represent the time sequence when pressures increases from diastolic minimum to systolic maximum, and the pressure change occurring during this time sequence is the amplitude. The maximum ($P_{max}$) and minimum ($P_{min}$) values for the single waves are identified, and the matrix computed containing the amplitudes ($\Delta P$) on the vertical column and latencies ($\Delta T$) on the horizontal row. Accordingly, the amplitudes are related to the naming of the columns and the latencies to the naming of the rows. The number or percentages of the single waves with the various combinations of amplitude and latency are computed within a first matrix. The balanced position of occurrences of amplitude ($\Delta P$) and latency ($\Delta T$) are determined. In a one-dimensional second matrix is plotted the number of occurrences of single pressure waves with a given rise time coefficient ($\Delta P/\Delta T$) plotted, and the balanced position determined. Furthermore, the absolute pressure values during said time sequence is determined, either as mean pressure for the whole time sequence or as mean pressure for the single pressure waves solely during said time sequence. All of these single pressure wave parameters related to a recording sequence may be stored in a database.

Single waves are recorded repeatedly during a fixed time sequence (e.g. each 5 seconds). Such selected time sequences may have various durations, preferentially between 5 to 15 seconds. The actual heart rate should not influence the results in this particular situation). Various types of on-line presentation are possible. When balanced position of amplitude ($\Delta P$) and latency ($\Delta T$) are plotted in a two-dimensional weighted matrix, the balanced position may be represented as one weighted value, e.g. in a trend plot (weighted value on the Y axis and time on the X axis), or in a histogram (weighted value on the X axis and proportion or absolute number of occurrences on the Y axis). For the given time sequence, the numbers or percentages of single wave combinations are presented within the histogram. On the Y-axis is indicated percentage occurrence of the various single wave combinations of latency and amplitude, and the various latency/amplitude combinations are indicated on the X-axis. For example, the bar within the histogram with label on the X-axis of 0.2|3.5 indicates the percentage occurrence of the single wave with a latency of 0.2 seconds and amplitude of 3.5 mmHg in percentage of the total numbers of single waves during the actual recording time of 5 seconds. The matrix and/or histogram may be subject to a number of statistical analyses. In one embodiment it is useful to determine the balanced position within the histogram or matrix. This balanced position may be termed the centroid or the centre of distribution, though these terms represent no limitation of the scope of the invention. In this invention the term balanced position is preferred. Balanced position may refer to the balanced position of occurrences of amplitude ($\Delta P$) and latency ($\Delta T$) combinations in the first matrix (see Table I), or to balanced position of rise-time coefficients ($\Delta P/\Delta T$) in the second matrix. In this situation, balanced position is the mean frequency distribution of the single pressure wave parameter combinations.

According to the invention the matrixes and histograms of single wave distribution may be computed repeatedly online including alarm functions for the single wave distributions that may be considered as abnormal. Thereby, continuous update of single waves is an alternative way of presenting pressures. In such an implementation single wave distribution may be updated each 5 or 10 seconds.

According to the invention, a method is described for more optimum analysis of pressure signals from detection of single pressure waves by means of non-invasive pressure sensors. During short time sequences of pressure recordings (e.g. each 3 seconds) the single pressure wave parameters are computed. Between each of said time sequences a sensor-regulating device is modified by a regulator providing a control signal to the sensor-regulating device. Results of said analysis within the processing unit provide a control signal to the regulator that in turn provide another control signal to the sensor-regulating device. Thereby, the inventive method of single wave analysis may modify the function of the sensor device to give the most optimal single pressure wave detection. An example is described with regard to applanation tonomtery, though this represents no limitation of the scope of the invention. A pneumatic pump and bellow press the transducer array against the skin and tissue above the cavity wherein pressure is measured (e.g. artery), usually referred to as the hold down pressure. In some devices the monitor searches through a range of pressure values until it measures an optimal signal in order to determine optimal hold-down pressure. Determining single wave distribution is however not possible by these methods. The present invention enables optimum single pressure wave detection. Furthermore, according to the present invention, there is no need for calibration by an independent technique. With regard to monitoring of fontanel pressure in infants basically the same principles are used. In these cases, tonometry enables pressures to be measured non-invasively on neonates. None of the techniques of tonometry provides the opportunity for sampling of single pressure waves.

The particular features of the invention are described in the attached independent method claims, whereas the related dependent claims describe advantageous, exemplifying embodiments and alternatives thereof, respectively.

## BRIEF DESCRIPTION OF THE DRAWINGS

FIG. 1 shows (a) parameters of a single pulse pressure wave, and (b) two time sequences wherein single pressure waves are identified.

FIG. 2 shows (a) one time sequence wherein all maximum and minimum values in the continuous pressure signal are detected, and (b) the identical time sequence wherein only the accepted minimum/maximum ($P_{min}$/$P_{max}$) pairs are shown.

FIG. 3 shows (a) one time sequence illustrating computation of absolute mean pressure for said time sequence according to two methods, and (b) relationships between absolute mean pressures computed according to the two methods for a large group of time sequences.

FIG. 4 shows (a) one time sequence wherein only false minimum/maximum ($P_{min}$/$P_{max}$) pairs are shown, and a trend plot of absolute mean pressure computed according to (b) the first or (c) the second method.

FIG. 5 shows two identical time sequences with identical time reference for continuous (a) arterial blood pressure and (b) intracranial pressure measurements.

FIG. 6 shows a differential plot of two simultaneous continuous pressure recordings with identical time reference with regard to (a) absolute mean pressure, (b) balanced position of amplitude, and (c) balanced position of latency.

FIG. 7 shows the scatter plots for determining the best fitted curves for the relationships between (a) balanced positions of amplitude and latency, (b) mean pressure and balanced position of latency, and between (c) mean pressure, balanced position of amplitude and balanced position of latency.

FIG. 8 shows a sequence of events during real time monitoring of single wave distribution, including (a) determination of single pressure waves within time sequences, (b) plotting combinations of single pressure wave amplitudes and latencies during said time sequence within a matrix, and (c) determining numerical value of balanced position of amplitude and latency within matrix. The figure as well shows presentations of weighted values of balanced position of amplitude and latency within time sequences within (d) histogram, (e) trend plot, and (f) pressure-volume curve.

FIG. 9 shows the pressure curves and histogram presentations of single wave distribution for intracranial pressure measurements within (a-b) the brain parenchyma and (c-d) the epidural space.

FIG. 10 shows two repeated pressure curves (a) before and (c) after pressure reduction, including accompanying histograms of single wave distribution (b) before and (d) after pressure reduction.

FIG. 11 shows trend plots of absolute mean intracranial pressure for two individuals (a, c), and trend plots (b, d) of weighted values (referred to as predicted mean pressure) of balanced position of amplitude and latency combinations within said time sequences.

FIG. 12 shows (a) an overview of a system for interaction between a processing unit, a regulator and a sensor-regulating device, for more optimum (b) single pressure wave detection during said time sequences, also indicating (c) modifications in control signal level applied to a sensor regulating device.

## DETAILED DESCRIPTION OF PREFERRED EMBODIMENTS

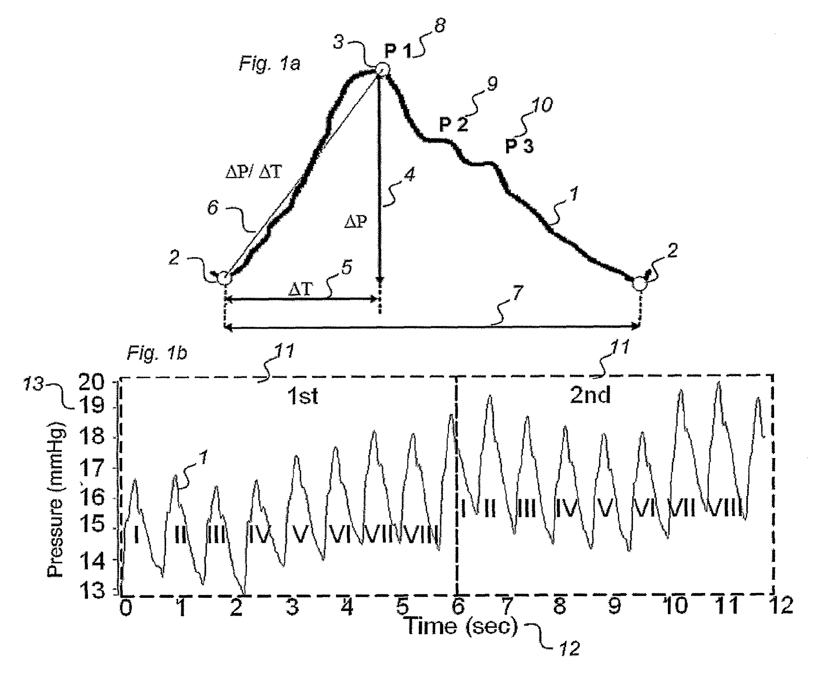

With regard to sampling, analysis and presentation of single pulse pressure waves 1, relative differences in pressures are computed, not related to a zero pressure level such as the atmospheric pressure. The invention provides measurement and analysis of the following parameters included in the time sequences (FIG. 1):

a) Minimum ($P_{min}$) 2 is defined as the diastolic minimum pressure of the single wave, or as the valley of the wave. An individual single pressure wave starts and ends with a diastolic minimum ($P_{min}$) value.
b) Maximum ($P_{max}$) 3 is defined as the systolic maximum pressure of the single wave, or defined as the peak of the wave.

c) Amplitude ($\Delta$P) **4** is defined as the pressure difference when pressures increase from diastolic minimum pressure ($P_{min}$) to systolic maximum pressure ($P_{max}$).

d) Latency ($\Delta$T) **5** is defined as the time interval of the single wave when the pressures change from diastolic minimum pressure ($P_{min}$) to systolic maximum pressure ($P_{max}$).

e) Rise time coefficient ($\Delta$P/$\Delta$T) **6** is defined as the relationship between amplitude divided by latency.

f) Wavelength **7** is defined as the duration of the single pulse pressure wave between the diastolic minimum pressure ($P_{min}$) **2** representing the start of the wave and the diastolic minimum pressure ($P_{min}$) **2** representing the end of the wave. Wavelength is referred to as $P_{min}$ - $P_{min}$ duration wherein first $P_{min}$ is inclusive and ending $P_{min}$ is not inclusive.

Whether the waveform is reproduced properly or not depends as well on a sufficient resolution order and a sufficient sampling rate. In FIG. **1a** is shown a standardized single pulse pressure wave 1, indicating the various parameters of a single wave that may be analyzed quantitatively. A single intracranial pressure wave may contain three peaks, the first (P1) **8,** second (P2) **9** and third (P3) **10** peaks. The maximum peak is termed the first peak (P1) **8** or top of the percussion wave. During the declining phase of the wave, there are two peaks namely the second peak (P2) **9,** often termed the tidal wave, and the third peak (P3) **10,** often termed the dicrotic wave. It is well known from the prior art that the absolute pressure value may determine whether the various peaks are present or not. The maximum value ($P_{max}$) **3** usually is related to the first peak (P1) **8,** but as pressure increases also the second peak (P2) **9** may become the maximum value ($P_{max}$) **3.** In this latter situation the systolic maximum ($P_{max}$) **3** is represented by the second peak (P2) **9.** It should be noted that the algorithm of defining single waves **1** according to the maximum ($P_{max}$) **3** and minimum ($P_{min}$) **2** values, it is not always possible to determine whether the maximum ($P_{max}$) **3** value represents the first (P1) or second (P2) peak. Not to introduce misunderstandings, the present invention relates to identification of single pressure waves **1** according to the systolic maximum ($P_{max}$) **3** and the diastolic minimum ($P_{min}$) **2** pressure values. Thus, in the present application, the amplitude ($\Delta$P) **4** of the first peak is defined as the pressure difference between the diastolic minimum pressure ($P_{min}$) **2** and the systolic maximum pressure ($P_{max}$) **3,** the latency of the first peak ($\Delta$T) **5** is defined as the time interval when pressures increases from diastolic minimum **2** to systolic maximum **3** pressures. It should be noted, however, that it is not always possible to exactly identify whether the top of the wave corresponds to the first peak or not. Whether or not the various peaks are identified depends on sampling rate and/or resolution. Therefore, the method described here does not require that the different peaks (P1-3) are identified, in other words the inventive method is not limited to the identification of the specific peaks P1 - P3. Thus, single waves **1** are identified according to diastolic minimum ($P_{min}$) **2** and systolic maximum ($P_{max}$) **3** values, independent whether systolic maximum ($P_{max}$) **3** is related to the first (P1) **8** or second (P2) **9** peaks. In practice it may be nearly impossible to differentiate the first and second peaks.

In another embodiment reference may be to the second (P2) **9** and third (P3) **10** peaks. The maximum and minimum values may be specified for the different peaks (P1 - P3), wherein also the single pressure wave parameters amplitude **4,** latency **5,** and rise time **6** coefficients are with reference to each of the specific pressure peaks (P1 - P3) **8-10.** In this situation, the identification of the first peak (P1) **8** is relative to maximum **3** and minimum **2.** The identification of the second peak (P2) **9** also is relative to the first peak (P1) **8,** and the third peak (P3) **10** is relative to the second peak (P2) **9.** This embodiment requires that each peak (P1 - P3) is determined within the single pressure wave 1. Thereby, latency **4,** amplitude **5,** and rise time coefficient **6** may be with reference to each of said single pressure waves.

An important inventive step is the identification of single pressure wave parameters within given time sequences **11.** A time sequence **11** refers to a specified time period of pressure recording during a continuous pressure monitoring. With reference to current technology absolute mean pressure of continuous pressure signals usually is computed within short time sequences **11** of 5-10 seconds. This is done because it is useful to update regularly the pressure values during a time of continuous pressure recording. In order to be comparable against current technology, the inventor select time sequences of between 5 and 15 seconds duration. Thus, it is suggested that the lengths of the time sequences should be in the range of 5-15 seconds. The inventor found it useful to use time sequences of 5 or 6 seconds. In the latter situation, a continuous recording period is considered as built up of a series of numerous continuous short time sequences of 6 seconds. The number of 6 seconds time sequences **11** are 10 during one minute, 300 during 30 minutes, 600 during 1 hours, 6 000 during 10 hours and 12 000 during 20 hours of continuous pressure recording. The inventor considered these 6 second time sequences **11** as the building blocks of the recording period. However, these suggestions of durations (e.g. 5 or 6 or 8 second durations) of time sequences **11** (or building blocks) should not be regarded as a limitation of the scope of the invention, as the time sequences might be of any duration selected by the user.

With reference to FIG. **1b,** on the x axis is indicated the time of pressure recording **12,** and on the y axis is indicated the pressure levels **13.** Along the time scale **12** two time sequences **11** each of 6 seconds duration are indicated. The first time sequence **11** (named 1st) lasts from seconds 1-6 on the time scale **12,** and includes eight single pressure waves **1** (numbered I-VIII). The second time sequence **11** (named 2nd) lasts from seconds 6-12 on the time scale **12,** and also includes eight waves **1** (numbered I-VIII). On the pressure scale **13** is indicated the absolute pressure levels, as well as giving an indication of the values of the single wave amplitudes **4.** As indicated in Fig. **1b,** the first single pressure wave **1** in the second time sequence **11** (named 2nd) is wave named I. This single wave **1** has its final minimum

($P_{min}$) **2** within the 2nd time sequence. The inventor applied the criterion that a time sequence **11** always starts with a single pressure wave **1** with complete wavelength **7** ($P_{min}$ - $P_{min}$), wherein the final $P_{min}$ is within said time sequence. When considering the 1st time sequence (named 1st), the final wave does not terminate within the 1st time sequence but terminates within the 2nd time sequence. Therefore, this wave is included in the 2nd time sequence. In order not to exclude single waves from the analysis, criteria must be included for determining whether a single pressure wave **1** located between two time sequences **11** should be located within the preceding or subsequent time sequence. The strategy relies on selected criteria. This strategy represents no limitation concerning the concept of time sequences.

The method of analysis of continuous pressure signals described in this invention is applied to continuous pressure signals during such selected time sequences. The method is applied to all continuous pressure signals for each of said time sequences in a continuous series of said time sequences during a continuous measurement period. Accordingly, a continuous pressure recording period is considered as built up of a continuous series of said time sequences wherein said time sequences are accepted or rejected for further analysis according to selected criteria.

Based on measurement of a continuous pressure signal, various strategies may be used to identify the single waves. Each pressure signal may be identified on the time scale **12** because pressures are recorded along with a time reference. In one implementation, single waves are identified according to the maximum ($P_{max}$) **3** and minimum ($P_{min}$) **2** values. The following is an example of the procedure of identifying maximum ($P_{max}$) **3** and minimum ($P_{min}$) **2** values, though the example is not intended to limit the scope of the invention.

The procedure of sampling signals indicative of pressure and converting said signals into digital data are described. The specific steps described here represent no limitation as several strategies may be used. The first part of the signal conditioning is the software filter. This filter removes a great deal of the high frequency noise. The source of the high frequency noise is not always possible to point out, but it will always be present in many different shapes and magnitudes. Various filters may be used. The inventor found it useful to apply a 25th order Bessel low pass filter, with a 25 Hz cut-off frequency. Other filters are available. The filter is programmed in a manner that removes both the transient part and phase lag. This is done by taking a copy of the first 100 samples in the signal, and then reversing the order. Then the copy of the first 100 samples is concatenated to the original signal. This process is also repeated for the 100 last samples in the signal. Then this signal is processed in the digital filter, the transient part will appear in the "new concatenated" part in the signal. It will not destroy the original signal. In order to remove phase lag, the filtered signal is restored by taking a subset of data from the signal processed by the filter algorithm. The subset is taken from sample index 109, with a length equal to the original signal. The specific values referred to in this paragraph depend on sampling frequency and other variables, and should not be regarded as limitations of the scope of the invention.

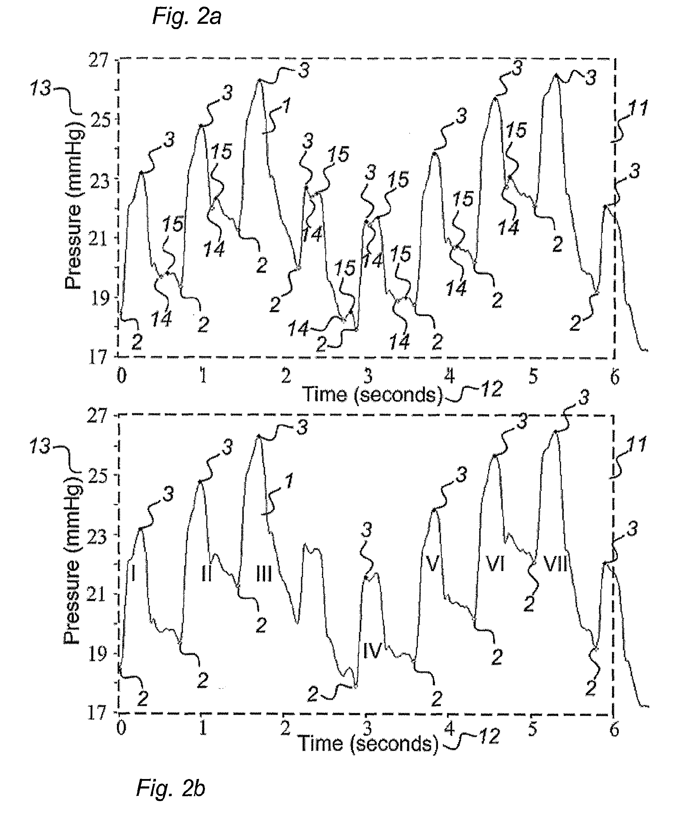

Reference is now given to FIG. 2. Most of the pressure signals in the human body are very dynamic signals, which contain a lot of peaks and valleys, not related to diastolic minimum and systolic maximum pressures. This paragraph describes the procedure of defining peaks related to systolic maximum pressure ($P_{max}$) **3** and valleys related to diastolic minimum pressure ($P_{min}$) **2**. It should be noted that determination of peaks ($P_{max}$) and valleys ($P_{min}$) may as well be related to the specific peaks (P1-P3) within said single pressure waves **1.** The signals are also sometimes garbled with artificial signals. In this context peaks refer to maximum values and valleys to minimum values. The result may be a lot of unwanted maximum ($P_{max}$) **15** and minimum ($P_{min}$) **14** detections. An unwanted or artificial minimum ($P_{min}$) **14** value is a minimum value that does not represent the diastolic minimum of said single pressure wave, and an unwanted or artificial maximum ($P_{max}$) **15** value does not represent the systolic maximum value of said wave. The peaks or valleys that are considered as unwanted or artificial depend on the criteria used during the identification procedure. As indicated in FIG. **2a,** the procedure of identifying maximum ($P_{max}$) **3** and minimum ($P_{min}$) **2** values always results in a lot artificial maximum ($P_{max}$) **15** and minimum ($P_{min}$) **14** detection values. In FIG. **2a** all the detected minimum and maximum values are shown. In other words the peak and valley detections have to be refined according to selected criteria. Therefore, wrong maximum **15** and minimum **14** values have to be removed. A total of eight wrong maximum **15** and eight wrong minimum **14** values are indicated in FIG **2a.** The acquired signal is first run through separate detection of minimum and maximum values. The maximum peak threshold value (or peak) is set to the lowest level in the signal, with duration longer than predefined values. A variety of pre-defined values may be chosen. The minimum threshold (or valley) is set to highest signal level, and the duration of the valley is a pre-defined value, as described above. Subsequent to this analysis, all maximum and minimum values are represented with an amplitude value and a location value or time stamp. This procedure will result in a lot artificial maximum ($P_{max}$) **15** and minimum ($P_{min}$) **14** detection values. Therefore the maximum and minimum detection has to be refined. After refinement, the result is a collection of approved maximum ($P_{max}$) **3** and minimum ($P_{min}$) **2** pairs (Fig. 2b) that may be presented to the function handling the dynamic parameter analysis. First, grouping of the maximum values and minimum values is performed. For every maximum the subsequent minimum is found. This couple makes a maximum-minimum ($P_{min}/P_{max}$) pair. The latter maximum-minimum pair is inspected for threshold level. The threshold value has to be larger than a given value. Subtracting the maximum amplitude and minimum amplitude performs this. The inventor found it useful to use the following criteria for intracranial pressure: Amplitude ($\Delta P$) **4** must be between 1.0 and 35.0 mmHg, and latency ($\Delta T$) **5** between 0.10 and 0.40 seconds. For arterial blood pressure, the thresholds were 30-120 mmHg for amplitude ($\Delta P$) **4** and 0.10 to 0.40 seconds for latency (AT) **5.**

These thresholds represent, however, no limitation of the scope of the invention. Other thresholds may as well be used. The pre-defined values may depend on age, and other variables such as type of pressure, type of cavity wherein pressure is measured, as well as underlying diseases. If the amplitudes ($\Delta P$) 4 and latencies ($\Delta T$) **5** are different from the pre-selected values, the pair is discarded. All the dynamic values are calculated by using the approved minimum-maximum ($P_{min}/P_{max}$) pairs. Only approved $P_{min}/P_{max}$ pairs are entered into the time sequences for further analysis. Thus, an accepted $P_{min}/P_{max}$ pair refers to an accepted diastolic minimum ($P_{min}$) **2** value followed by a subsequent systolic maximum ($P_{max}$) **3** value, indicative of an accepted single pressure wave **1**. Criteria are applied as well to which diastolic minimum ($P_{min}$) **2** value that is considered to terminate the single pressure wave. The values which are calculated are amplitude ($\Delta P$) (delta intracranial pressure expressed in mmHg) **4**, latency ($\Delta T$) **5**, rise time coefficients ($\Delta P/\Delta T$) **6**, and heart rate **16**. The latency ($\Delta T$) **5** from minimum to maximum is the time where the pressure of the single wave increases from the diastolic minimum pressure ($P_{min}$) **2** to the systolic maximum pressure ($P_{max}$) **3**. Afterwards the $P_{min}/P_{max}$ pair is inspected for the $\Delta P/\Delta T$ **6** value. The $\Delta P/\Delta T$ **6** value can be expressed as (peak amplitude- valley amplitude) divided by (peak location-valley location). This will further remove $P_{min}/P_{max}$ pairs caused by for example an artefact in the collected signal. All $\Delta P/\Delta T$ values with a value equal or larger than a given value are discarded. Another criterion is related to the wavelength duration. Since the wavelength is a measure of the heart rate, the heart rate represents still another criterion. After applying the various criteria to single pressure wave detection, the collection of peaks and valleys now contains only approved $P_{min}/P_{max}$ pairs, corresponding to approved single pressure waves.

With reference to FIG. **2b**, a total of 8 accepted $P_{min}/P_{max}$ pairs are indicated. The individual single pressure waves **1** are indicated along with the time reference on the time scale **12**, and the levels of the single wave amplitudes are indicated on the pressure scale **13**. The duration of said time sequence is 6 seconds. It is indicated that each of these $P_{min}/P_{max}$ pairs (i.e. single pressure waves) have a diastolic minimum **2** value followed by a subsequent maximum **3** value. Furthermore, the relation of the single pressure waves **1** to the time sequence **11** is indicated. The first 7 accepted $P_{min}/P_{max}$ pairs correspond to the first seven single pressure waves (waves named I, II, III, IV, V, VI, VII). The final accepted $P_{min}/P_{max}$ pair has no number since this wave is not included in this time sequence **11**. The reason for this is that no accepted minimum **2** value was identified within the time sequence **11**. Provided that such an accepted minimum **2** value is determined within the subsequent time sequence, this wave will become the first wave in the following time sequence (see FIG. **1b**).

Thus, during a given recording period all single pulse pressure waves are identified. However, due to artifacts some waves are missed. The software allows the computation of numbers of artifacts and missed single waves, as well as relates this to total counts of single waves. Hence, the artifact ratio may be computed. Given that the numbers of artifacts are considered as too high a recording period may be omitted from analysis. Such artifacts relate to pressure recording sequences without accepted single pressure waves (i.e. accepted $P_{min}/P_{max}$ pairs). There are several reasons for not identifying single pressure waves: Failure of pressure sensor may cause erroneous pressure recordings. Noise in pressure signals is another reason. The identification of the correct single pressure waves provides the opportunity for only including those parts of the pressure recordings that include single pressure waves.

Measurement of single waves requires a continuous pressure signal, though the pressure signals may be sampled at a variable rate. The sampling frequency preferably should be above 10 Hz. The inventor initially found it sufficient to use a sampling rate of at least 100 Hz to identify maximum ($P_{max}$) **3** and minimum ($P_{min}$) **2** values. A higher sampling rate (at least 200Hz) may be required to find the maximum $P_{max}$ **3** and minimum $P_{min}$ **2** values for the individual peaks (P1 - P3) **8-10**. When valleys ($P_{min}$) and peaks ($P_{max}$) are determined with reference to the specific peaks P1-3, said valleys and peaks are relative to maximum values ($P_{max}$) related to systolic maximum pressure and minimum values ($P_{max}$) related to diastolic minimum pressure for said single pressure waves.

The invention is not limited to a particular range of sampling frequencies. Rather the sampling rate should be sufficient to detect the various single pressure wave parameters (i.e. $P_{min}$, $P_{max}$, $\Delta P$, $\Delta T$, and $\Delta P/\Delta T$).

In summary, the procedure of identifying correct $P_{min}/P_{max}$ pairs includes different steps: (1) Filter and concatenation of digital pressure signals. (2) All minimum ($P_{min}$) and maximum ($P_{max}$) values are identified and represented with an amplitude value and a location value (or time stamp). (3) All $P_{min}/P_{max}$ pairs are identified, where the subsequent minimum ($P_{min}$) value is found for every maximum ($P_{max}$) value. (4) Only those $P_{min}/P_{max}$ pairs meeting certain pre-selected criteria concerning thresholds for $\Delta P$, $\Delta T$ and $\Delta P/\Delta T$ are accepted. (5) The single pressure wave parameters for given time sequences are determined. (6) The time sequences are subsequently accepted or rejected, according to criteria for the time sequences.

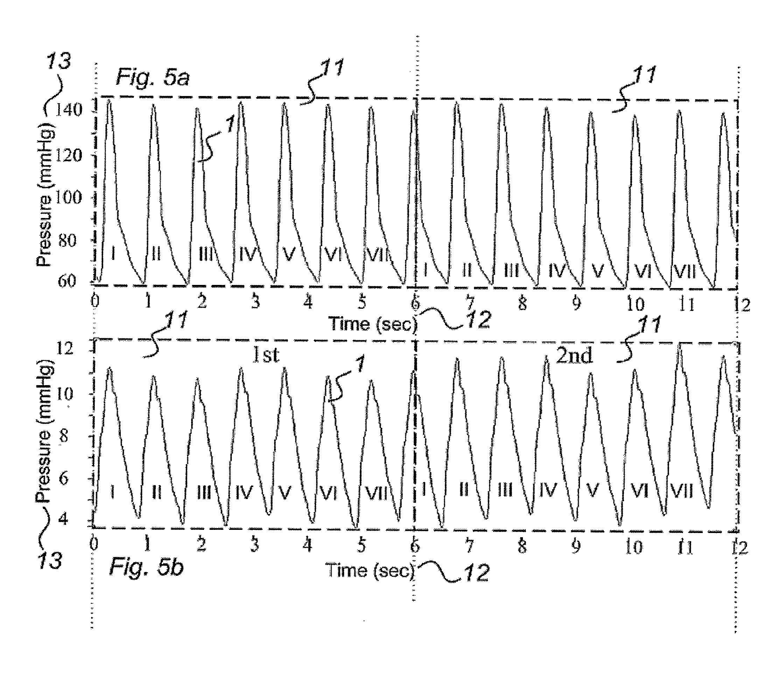

With reference to the time sequences **11**, a question is which single pressure waves that should be included. A time sequence may contain parts of single waves both in the first and final part of the time sequences. It is not useful to discard waves of this reason. During a continuous recording period, single pressure waves **1** occurring between two time sequences are included in the first or second time sequence according to selected criteria. The invention is not limited to which criteria that are used. The inventor used the following procedure: First, with regard to the final part of a time sequence **11**, the inventor found it useful to include in the time sequence **11** the single wave **1** that is terminating within the time sequence **11**, which is the single wave terminating with its last $P_{min}$ within said time sequence. Thus, the last single pressure wave 11 included within a time sequence **11** will have its whole wavelength ($P_{min}$ - $P_{max}$ - $P_{min}$) within that particular time sequence, including its last $P_{min}$. As indicated in FIG. **2b,** the accepted $P_{min}/P_{max}$ pair subsequent to single wave no. VII is not included in the time sequence presented. If the last accepted $P_{min}/P_{max}$ pair has no $P_{min}$ the inventor found it useful to use this wave in the following time sequence, provided a following time sequence is measured. Thereby, the single pressure waves may be immediately analysed for said time sequence without waiting for the results of analysis of the next time sequence. Second, when considering the first part of a time sequence **11,** the first single wave 1 will have its final $P_{min}$ within this time sequence. With reference to FIG. **1b,** in the second time sequence (named 2nd) the first wave (named I) is the single wave with its final $P_{min}$ within the second time sequence. The same aspect is indicated in FIGS. **5a** and **5b.**

In FIGS. **1b, 2a** and **2b** are indicated on the pressure scale **13** (y axis) the absolute pressure levels, which also give an indication of the values of amplitudes ($\Delta P$) **4** of the single pressure waves **1.** The amplitude ($\Delta P$) **4** values are relative values, not related to a zero pressure level, since the amplitude ($\Delta P$) **4** levels represent the pressure difference between the systolic maximum ($P_{max}$) **3** and diastolic minimum ($P_{min}$) **2** pressure levels. This is an important aspect of the invention.

The absolute pressure levels of the single pressure waves 1 may as well be determined. The pressure scales **13** of FIGS. **1b, 2a** and **2b** refer to the absolute pressure levels that are relative to the atmospheric zero pressure level. The term absolute pressure refers to the situation when pressure is relative to the atmospheric zero pressure level. According to current technology, absolute pressures may be computed as mean or average of pressures during a time sequence of 5 seconds, and shown on the Y-axis. The X-axis shows the time of pressure recording. The fundamental difference between the curve according to the present invention and the conventional curve according to current technology relates to the strategy of processing pressure signals. According to current technology, pressures may be processed in sequences of 5 seconds, and the mean or area under curve for continuous pressure signals during the 5 seconds period is computed. By this conventional approach information about single waves are missed. The time period wherein mean pressure is computed may vary and depending on the monitor system. Furthermore, there is a wide range concerning pressure sampling frequency. Most monitors compute mean pressure in sequences of 5-8 seconds, though the term absolute mean pressure does not refer to a particular recording period.

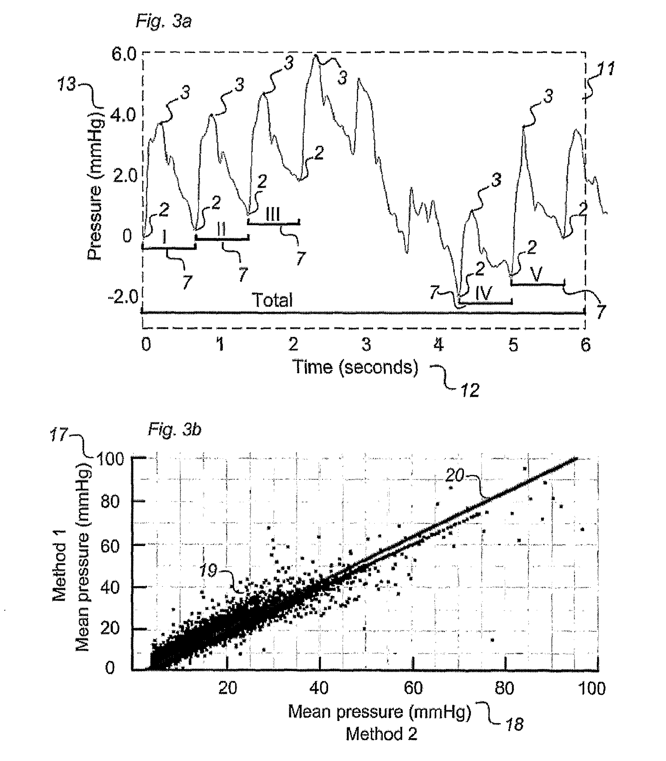

This invention introduces a new inventive step concerning computation of absolute mean pressure, related to time sequence. This method is hereafter referred to as Method 2, as opposed to Method 1, representing conventional or current technology. The differences between conventional technology (Method 1) and the method according to this invention (Method 2) are illustrated in FIG. **3a.** Given that absolute mean pressure is computed for a time sequence 11 of 6 seconds, current technology takes into account all recorded pressure signals during the time period, indicated by the line termed Total in FIG. **3a.** Absolute mean pressure for said time sequence **11** is the sum of all sample values (pressure levels) divided by numbers of samples during said time sequence. In equation 1 is shown the procedure of computing mean pressure (x=pressure level for each sample, and n= numbers of samples in the sequence). According to Method 1, a absolute mean pressure of 1.85 mmHg was computed for the time sequence presented in FIG. **3a.**

$$Mean = \frac{1}{n} \sum_{i=0}^{n-1} x_i \qquad \ldots\ldots\ldots(1)$$

The time sequence indicated in FIG. **3a** shows five accepted single pressure waves **1** within said time sequence (named I, II, III, IV, and V). According to the criteria used, the pressure signals between single wave III and IV were not indicative of single pressure waves. The basic concept of this invention is that pressure values are only relevant when related to single pressure levels, since pressure signals not indicative of single pressure waves probably represent noise, not related to pressure *per se.* Mean pressures computed according to conventional technology does not take into account whether pressure signals are related to single pressure waves or not. This invention introduces a new inventive step, namely computing mean pressure for said time sequence **11** as the sum of mean pressure for all individual single pressure waves ($P_{min}$ to $P_{min}$) divided by the number of waves during said time sequence. Thereby, pressure samples not related to single pressure waves are not included in determining absolute mean pressure for the particular time sequence. For the time sequence presented in FIG. 3a the wavelength ($P_{min}$ to $P_{min}$) **7** for each individual single wave (I to V) is indicated. In this situation the Formula 1 is applied to each individual single wave, wherein each wave begins and ends with a diastolic minimum value, equal to the wavelength ($P_{min}$ to $P_{min}$) **7** of said single pressure wave **1.** According to equation 1, for each individual single wave, the sum of all pressure samples (pressure levels) is divided by the numbers of samples. In this particular example, the sampling rate was 100 Hz. Absolute mean pressure was 2.5 mmHg for single pressure wave I, 2.27 mmHg for wave II, 2.96 mmHg for wave III, -0.45 mmHg for wave IV and 1.07 mmHg for wave V. Second, for the whole time sequence, the sum of mean pressure for each individual single pressure wave during said time sequence is divided by the number of single waves. Non-accepted single waves (or time sequences) are not included in the analysis.

Mean pressure for the time sequence in FIG. **3a** according to Method 2 was 1.67 mmHg [(2.5+2.27+2.96-0.45+1.07)/5]. Actually the difference was small concerning absolute mean pressure computed according to Method 1 (1.85 mmHg) and Method 2 (1.67 mmHg).

In FIG. **3b** is shown the relationships between absolute mean pressure computed according to Method 1 or Method 2. On the y axis, the absolute mean pressure 17 refers to mean pressure computed according to Method 1, with reference to absolute mean pressure **18** computed according to Method 2 on the x axis. Each plot in the scatter **19** refers to absolute mean values computed by either of the methods. Both methods were applied to individual time sequences of 6 seconds during a series of continuous time sequences in a total of 75 continuous pressure recordings. These 75 continuous pressure recordings included a total of 873546 time sequences **11,** each lasting 6 seconds. Of these time sequences, a total of 144835 time sequences were rejected according to selected criteria. No single pressure waves were found in 20862 time sequences. Thereby, the plot presented in FIG. **3b** is based on a total of 707849 time sequences, each lasting 6 seconds. As indicated in FIG. **3b**, at the group level a very high correlation exists between mean pressure computed according to Method 1 and Method 2, as indicated by the regression line **20.**

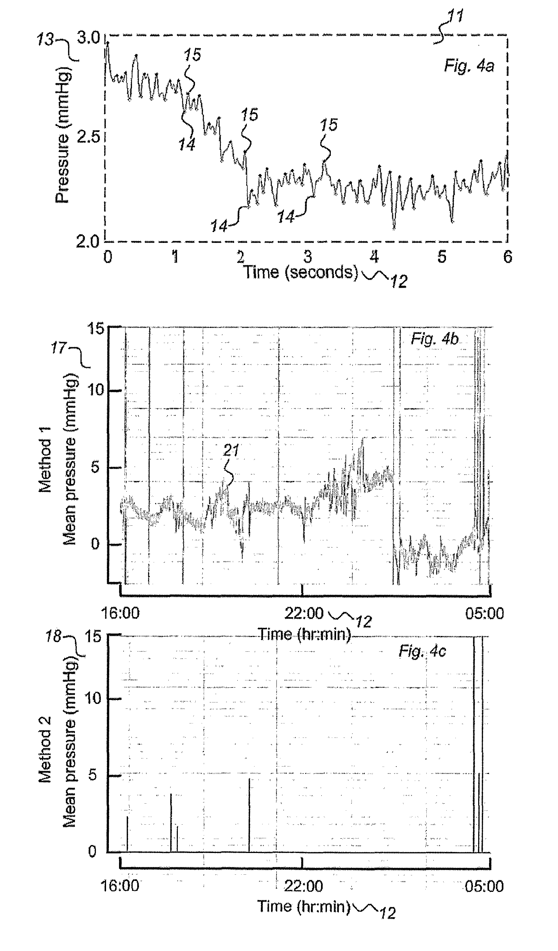

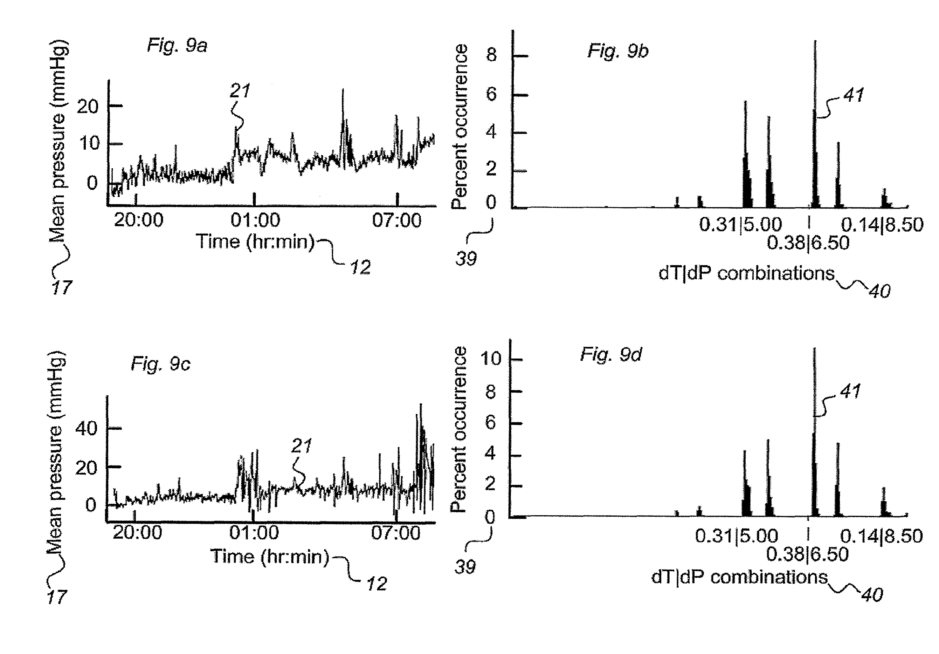

Despite a very high correlation between absolute mean pressures computed by either of the methods, a major advantage with the inventive method (Method 2) is that absolute mean pressure is computed only when single pressure waves are identified. If no single pressure waves are accepted or identified, no absolute mean pressure is computed. Method 1, on the other hand, computes mean pressure whether single pressure waves are present or not. Reference is now given to FIG. 4 that shows an intracranial pressure recording lasting about 11 hours and 54 minutes. This continuous recording period consisted of a continuous series of 7145 time sequences **11.** Only a total of 7 time sequences **11** were accepted, whereas 7134 time sequences 11 contained no single pressure waves, and 4 time sequences **11** were rejected according to selected criteria. In FIG. **4a** is provided an example of one of the time sequence **11** of 6 seconds that contained no single pressure waves. The absolute pressure levels were between 2 and 3 mmHg. As shown the pressure signal contained only unwanted or artificial minimum **14** and maximum **15** values, not related to single pressure waves 1. According to Method 1, mean pressure for each time sequence **11** is computed as the sum of all pressure sample levels divided by the number of pressure samples. In FIG. **4b**, the mean pressure **17** according to Method 1 computed within each time sequence each 6 seconds is plotted repeatedly against time in the time scale **12.** The mean pressure trend plot (pressure curve) 21 consists of repeated plots, wherein each plot represents the mean pressure value of the 6 seconds time sequence **11.** When only considering the pressure curve 21, it is not possible to know whether the curve is acceptable or not. Examples of absolute mean pressure trend curves **21** are also shown in FIGS. **9a, 9c, 10a, 10c, 11a,** and **11c.** In FIG. **4c** is shown another trend plot wherein the y axis shows absolute mean pressure computed according to Method **2 18** and the x axis the time scale **12.** No pressure curve was found in FIG. **4c.** Thus, computation of mean pressure within given time sequences according to the method described here (Method 2) gives the major advantage of not computing pressure values when single pressure waves are not identified. When single pressure waves are present, the method gives absolute mean pressure values very similar to the absolute mean pressures computed according to conventional technology.

Reference now is given to FIGS. **5a** and **5b.** One single wave **1** has the duration from one minimum value ($P_{min}$) back to another minimum value ($P_{min}$), which is the duration of the wave (see FIGS. **1a** and **3a**). During a time sequence **11,** the heart rate 16 may be computed according to two methods (HR-methods 1 and 2). According to the first method (HR-method 1), heart rate **16** is defined as equal to the number of single pressure waves during said time sequence, divided with the duration of said time sequence. With reference to FIG. **5a,** heart rate is equal to a number of waves divided by recording time (seconds). During the first time sequence **11** of 6 seconds 7 single pressure waves were identified (I to VII), giving a heart rate of 7/6 seconds (=1.2/second).

Another strategy (HR-method 2) is defining heart rate as numbers of waves divided by the total wavelength of said single pressure waves. In one single pressure wave, the entire wavelength is defined as the duration from $P_{min}$ to $P_{min}$. The heart rate is the number of single pressure waves during a time sequence divided by the duration of the time sequence wherein these waves occurs. Heart rate method 2 is somewhat more accurate than Heart rate method 1, since the first method only includes the duration of the time sequence wherein single waves occurred. With reference to FIG. **5a,** the sum of wavelengths (i.e. $P_{min}$ - $P_{max}$ - $P_{min}$) of the 7 single pressure waves (I to VII) were 5.7 seconds, heart rate would be 7/5.7 seconds (=1.2/second). Some minor differences in heart rate may be computed by the two methods.

The invention includes through its methods of analysis at least two levels of verifying whether a time sequence **11** includes correct single pressure waves: (1) Criteria for accepting or rejecting single pressure waves entering into the time sequences. (2) Criteria for accepting or rejecting the individual time sequence. If the time sequence **11** is not accepted according to the criteria, the time sequence **11** is rejected for further analysis.

First, the main strategy for identification of single waves 1 are related to criteria applied to accepted $P_{min}/P_{max}$ pairs concerning ranges for amplitude ($\Delta P$) **4,** latency ($\Delta T$) **5,** and rise-time coefficients ($\Delta P/\Delta T$) **6.** Single pressure waves not meeting the pre-selected requirements are rejected. A new time sequence **11** is started including the first accepted

$P_{min}/P_{max}$ pair, which is the first accepted $P_{min}/P_{max}$ pair that has its final $P_{min}$ **2** within that particular time sequence **11**. This has been commented on for FIGS. **2a** and **2b**. In FIG. **2a** is indicated a total of 17 $P_{min}/P_{max}$ pairs. After the single pressure wave criteria were applied to these 17 $P_{min}/P_{max}$ pairs, only a total of 8 $P_{min}/P_{max}$ pairs were accepted (FIG. **2b**). However, only 7 single pressure waves (I to VII) were included in the time sequence, as the accepted $P_{min}/P_{max}$ pair following wave VII was not followed by an accepted $P_{min}$ value within the particular time sequence. Therefore, this latter accepted $P_{min}/P_{max}$ pair was included in the subsequent time sequence, provided that an acceptable $P_{min}$ was identified.

Second, criteria may be applied to the individual time sequences **11,** determining whether the entire time sequence is accepted or not. The strategy is related to the numbers of single pressure waves (or the heart rate) during the time sequence. During a time sequence the heart rate **16** should be within physiological limits. Ranges for numbers of single waves within a time sequence may be defined. The inventor found it useful to define that the heart rate should be 40-180 (i.e. 4 to 18 single waves within a time sequence of 6 seconds). Thus, time sequences of 6 seconds duration containing a number of single pressure waves outside 4-18 are not accepted for further analysis. Other criteria may as well be used. In addition, thresholds may be defined for accepted variation of numbers of single waves within a time sequence. For several time sequences, standard deviation of numbers of single waves within the time sequence may be computed, wherein time sequences with numbers of single waves deviating too much from standard deviations are rejected.

When several pressures are monitored simultaneously with identical time reference, numbers of single pressure waves within identical time sequences may be compared, as illustrated in FIGS. **5a** and **5b.** For example, simultaneous monitoring of continuous arterial blood pressure (ABP) (FIG. **5a**) and intracranial pressure (ICP) (FIG. **5b**) with the same time reference provides the opportunity to compare numbers of waves for these two pressures during identical time sequences. During a given time sequence, numbers of single waves of ICP ($N_{SW-ICP}$) waves should be nearly equal to numbers of single waves of ABP waves ($N_{SW-ABP}$) [$N_{SW-ABP}$ - $N_{SW-ICP}$ < 2]. In the same way, heart rate (HR) derived from single waves of ICP (or CSFp) ($HR_{ICP}$) may be compared with heart rate (HR) of ABP ($HR_{ABP}$). During this recording period, the difference of heart rate (HR) derived from these pressures should be less than 2 [$HR_{ABP}$ - $HR_{ICP}$ < 2]. As illustrated in FIGS. **5a** and **5b,** the first time sequence (named 1$^{st}$) contains seven single pressure waves (named I-VII), identical to the second time sequence (named 2$^{nd}$). It should be noted that the numbers of single pressure waves were equal (nos. I-VII) for both arterial blood pressure (FIG. **5a**) and intracranial pressure (FIG. **5b**). The specific numbers (<2) are only for illustrative purposes and should not be regarded as limitations of the invention.

Comparisons between numbers of waves for one pressure type within a time sequence also may be compared against heart rate measurement from another source, for example pulse oxymetri ($spO_2$), or electrocardiography (ECG). Heart rate **16** during a given recording period derived from either arterial blood pressure or intracranial pressure waves should be equal to heart rate derived from oxygen saturation measurements or by electrocardiography (ECG) [$HR_{P-O2}$ - $HR_{ICP}$ < 2; $HR_{ECG}$ - $HR_{ICP}$ < 2]. The inventor found it useful to use criteria for differences in single waves <2, though other criteria may as well be applied.

For each time sequence **11,** all the single waves and single wave parameters are known. Accordingly, standard deviations for all the single pressure wave parameters may be computed. Standard deviations for relative pressures include the parameters amplitude ($\Delta P$) **4,** latency ($\Delta T$) **5,** and rise time coefficient ($\Delta P/\Delta T$) **6** for all single pressure waves 1 within the time sequences **11.** Standard deviations for absolute pressures include absolute pressure values such as mean pressure for all individual single pressure waves (i.e. mean pressure from $P_{min}$ to $P_{min}$ for each individual of all single waves within the time intervals), standard deviations for diastolic minimum ($P_{min}$) **2** for all individual single pressure waves during said time sequence, standard deviations for systolic maximum pressure ($P_{max}$) **3** for all individual single waves **1** during the time sequence **11.** Other criteria for acceptance or rejection of a time sequence may be related to limits for the standard deviations referred to here.

Repeated up-dates are made concerning numbers/proportion of accepted and rejected time sequences (and single waves). A log is made for numbers of rejected time sequences and numbers of rejected single pressure waves. A log also is made for reasons of rejection, such as abnormal $\Delta P$ **4**, $\Delta T$ **5**, $\Delta P/\Delta T$ **6**, or abnormal changes in HR **16**. Rejected portions of trend plot may be indicated by color of graph or background. Examples of such statistics were made for FIGS. **3b** and **4a.**

After identification of the single pulse pressure waves **1** during a recording period, the single pressure waves are subject to analysis. Fundamental to the invention is the computation of a matrix **36** of numbers or percentages of single pulse pressure waves with pre-selected wave characteristics. Examples of such characteristics are latencies ($\Delta T$) **5** and amplitudes ($\Delta P$) **4.** The matrix **36** of amplitude ($\Delta P$) **4** and latency ($\Delta T$) **5** combinations are referred to as the first matrix in this document. Again, the latencies and amplitudes in the matrix presented in Tables I, V and VI refer to single waves identified by diastolic minimum ($P_{min}$) **2** and systolic maximum ($P_{max}$) **3** values. As indicated, the group of amplitudes are shown on the horizontal row, and the grouping of the latencies on the vertical column. Each number **37** within the cells of said matrix **36** represents the total numbers of single waves with the given combination of amplitude **4** and latency **5.** In another situation the numbers **37** may refer to percentages. When percentages are used, it is against total numbers of waves. The amplitudes 4 are usually expressed in mmHg and the latencies **5** in seconds. The numbers of cells in such a matrix **36** may differ depending on the number of columns and rows. An example is given based on experience of the inventor. For intracranial pressure, the inventor found it useful to use the range of amplitudes (ΔP) **4** equal to 0 to 30.0 mmHg, with intervals of 0.5 mmHg, giving a total of 60 columns. The range of latencies (ΔT) **5** is 0.10 seconds to 0.40 seconds with intervals 0.01 seconds, giving a total of 30 rows. In this matrix the total cell number is 1800. The example represents no limitation of the scope of the invention. For arterial blood pressure, on the other hand, the inventor used the range of amplitudes (ΔP) from 30 to 120 mmHg, with intervals of 2.0 mmHg, giving a total of 45 columns. The range of latencies (ΔT) is 0.10 seconds to 0.40 seconds with intervals 0.01 seconds, giving a total of 30 rows. In this matrix the total cell number is 1350. However, these are only examples, and are not intended to limit the scope of the invention.

An example of a small part of a matrix applied to intracranial pressure is shown in Table I. The matrix **36** (referred to as first matrix) illustrate only a small fraction of a large matrix of 1800 cells.

Table I. Part of a matrix of amplitude (ΔP) and latency (ΔT) combinations.

| Group name | | | 0.5 | 1 | 1.5 | 2 | 2.5 |
|---|---|---|---|---|---|---|---|
| | Group range | | 0.5≤dP<1.0 | 1.0≤dP<1.5 | 1.5≤dP<2.0 | 2.0≤dP<2.5 | 2.5≤dP<3.0 |
| | | Group midpoint | 0.75 | 1.25 | 1.75 | 2.25 | 2.75 |
| 0.1 | 0.10≤dT<0.11 | 0.105 | | | | | |
| 0.11 | 0.11≤dT<0.12 | 0.115 | | | | | |
| 0.12 | 0.12≤dT<0.13 | 0.125 | | 3 | 12 | | |
| 0.13 | 0.13≤dT<0.14 | 0.135 | | 16 | 12 | 8 | |
| 0.14 | 0.14≤dT<0.15 | 0.145 | | 7 | 5 | 4 | |
| 0.15 | 0.15≤dT<0.16 | 0.155 | | | | | |

The amplitude (ΔP) **4** values are presented in the columns and the latency (ΔT) **5** values in the rows. For example the first column corresponds to the first amplitude (ΔP) group, named 0.5 (corresponding to 0.5 mmHg); this group includes amplitude (ΔP) **4** values greater or equal to 0.5 mmHg, but less than 1.0 mmHg (indicated by the group range 0.5≤ΔP<1). The midpoint (or mean) of the group is 0.75 [(0.5+1.0)/2]. Similarly, the first latency group is termed 0.1, corresponding to a latency of 0.1 seconds. This latency group includes latencies with a duration greater or equal to 0.10 seconds, but less than 0.11 seconds (indicated by the group range 0.10≤ΔT<0.11). The group midpoint is 0.105 [(0.10+0.11)/2]. The amplitude/latency (ΔP/ΔT) matrix can be seen as a two dimensional collection of bins, where the rows are labelled ΔT and the columns are labelled ΔP. A cell equals a bin. Each bin denotes how often ΔP/ΔT combinations have appeared. When the observation is categorized or grouped, the midpoint of the group is used. The data are categorized when the data are in a "range". As an example the first bin in the matrix presented in Table I contains all ΔP values which fall in the range greater or equal to 0.5 mmHg and less than 1 mmHg, with a ΔT value greater or equal to 0.10 seconds and less than 0.11 seconds. The matrix cells found at intersections between columns and rows indicate numbers or proportions of single pressure waves with specific combinations of amplitude (ΔP) and latency (ΔT). The numbers presented in Table I refers to an intracranial pressure recording lasting one minute including 10 time sequences **11,** each lasting 6 seconds. During these 6 time sequences a total of 67 single pressure waves occurred. The distribution of the various single pressure waves during this recording period is shown in Table I. For example, single pressure waves **1** with an amplitude (ΔP) **4** greater or equal to 1.5 mmHg but less than 2.0 mmHg and a latency (△T) greater or equal to 0.14 seconds, but less than 0.15 seconds occurred 5 times during the time sequence represented in this matrix.

During real time monitoring, the matrix may be computed each 5 seconds. The centre of mass of distribution (balanced position) of single waves with combinations of latency and amplitude may be computed, without taking into account the heart rate. Another implementation may be continuous update of single wave distribution each 5 or 10 seconds. On the monitor display a histogram is presented each 5 seconds in many monitors, though there may be differences between the monitors. The invention does not give any limitations concerning the frequency of updates of matrix.

The preferable approach suggested by the inventor, is repeated computation of the matrix during a continuous pressure monitoring. For each of said time sequences **11** the matrix **36** is computed. For example, with reference to FIG. **5a,** one matrix is computed for the first time sequence (named 1st) and a new matrix computed for the second time

sequence (named 2nd). For the first time sequence **11** (termed 1st), the matrix 36 contains the amplitude (ΔP) **4** and latency (ΔT) **5** values of seven single pressure waves. For the second time sequence **11** (termed 2nd), a new matrix **36** is computed also containing seven single pressure waves. For each individual time sequence, new ΔP/ΔT combinations are subsequently entered into the matrix cells during the ongoing pressure measurement. The matrix is updated dynamically during such a 6 seconds time sequence, each cell is updated by adding the new value to the old content. After the 6 seconds interval the procedure is reiterated starting with a new and empty matrix.

The matrix **36** presentations may be subject to various types of analyses. The balanced position within the matrix may be presented as numerical value combinations **38** such as centroid or centre of distribution. According to the present invention, the single waves **1** with pre-selected characteristics of latency **5** and amplitude **4** are computed, and the matrix **36** of single wave combinations computed, with presentation of the distribution of single wave combinations. For the 5 second period the balanced position of single wave combinations may be computed, for example as centroid or centre of distribution. For example, a combination **38** of 0.17|2.0 refers to the single wave combination of latency of 0.17 seconds and amplitude of 2.0 mmHg. During real-time monitoring these numerical value combinations may be updated each time sequence **11** of 5 seconds. In one embodiment, the numerical value combinations **38** may be presented on the display of the apparatus, though this is no limitation of the scope of the invention. For example, presentation on the display of monitoring systems is possible.

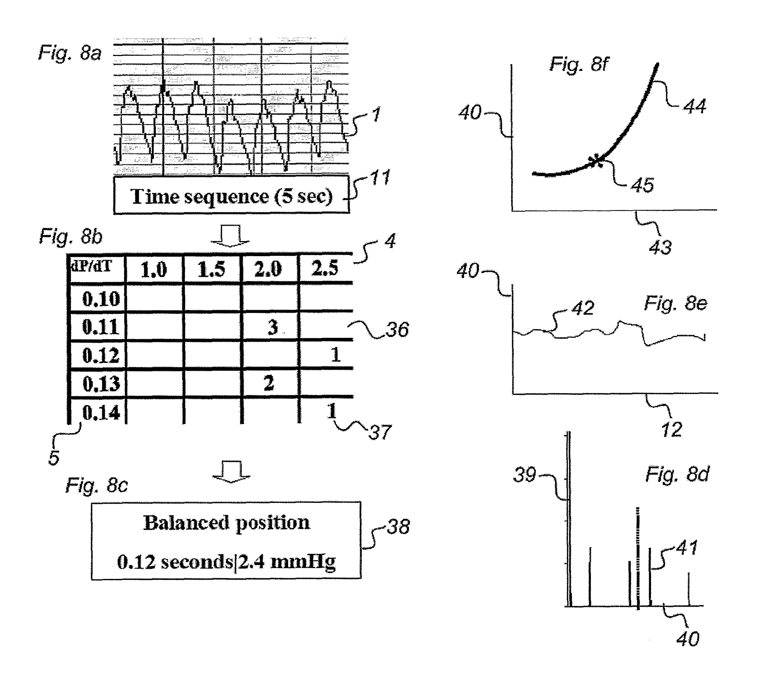

The balanced position of single wave combinations, for example determined as centroid or centre of distribution, may also be presented on the y axis in a xy chart with time on the x axis (see FIG. 8e). For example, the centroid or centre of distribution of single wave combinations may be computed repeatedly each 5 second and plotted in the XY-chart during a period of recording. In this situation, the curve reflects numerical value combinations of centroid or centre of distribution of single wave combinations within the histogram or matrix.

Balanced position within a matrix may have different names, such as centroid, centre of distribution, or centre of mass. In this document it is preferred to use the term *balanced position.* In the context described here, balanced position refers to mean frequency distribution of occurrences of single pressure wave parameters. In this document the terms balanced position of amplitude (ΔP), balanced position of latency (AT) and balanced position of rise time coefficient (ΔP/ΔT) are used as terms with respect to the first and seconds matrixes, respectively. However, the term balanced position *per* se is a method for determining the mean occurrence either within a one- or two-dimensional matrix in general.

With reference to the matrix presented in Table I, the procedure of computing balanced position of the distribution of different amplitude (ΔP) and latency (ΔT) combinations is described. The numbers referred to in Table I relates to a recording period of 1 minute. The method is however, similar whether the recording period is 5, 6 or 10 seconds, or 1 minute or 10 hours. The balanced position relates to the frequency distribution of the different occurrences of amplitude (ΔP) 4 and latency (ΔT) 5 during the selected time period. The method is similar independent on factors such as type of pressure, group ranges, or numbers of cells. Depending on the range of amplitude and latency values, the matrixes may contain a variable number of columns and rows. However, the balanced position result is dependent on the matrix resolution.

With reference to the matrix presented in Table I, the columns refer to amplitude (△P) **4** groups and the rows to the latency (△T) **5** groups. The system will have i rows, and *j* columns. When computing balanced position/centroid/ mean frequency of such a two-dimensional distribution (referred to as first matrix), both dimensions have to be considered. Since there are two variables the mean (or balanced position) must be given by two numbers, like the ΔT|ΔP values. With reference to Table I, if the values from the row and column mean are considered, the results may be interpreted as the mean value for the distribution. The mean distribution (or balanced position) for the numbers presented in Table I, is located in the crossing point between the two lines ΔP=1.64 and ΔT=0.135. This is the most accurate way to describe the balanced position (or mean value) for this two dimensional distribution. The balanced position in the matrix shown in Table I is ΔP=1.64 and ΔT=0.135, corresponding to the cell with count 12. In the following, some details are given concerning computation of mean row and mean column values. First, the latency (ΔT) mean value (or row mean), with respect to the amplitude (ΔP) values (columns) is determined. The m; for each latency (ΔT) row is determined, by using the equation 2.

$$m_i = \sum_{j=1}^{j=c} A_j w_{ij} \quad \ldots\ldots\ldots\ldots\ldots\ (2)$$

where $A_j$ is the j$^{th}$ column midpoint, referring to an amplitude (ΔP) group value; $w_{ij}$ is the frequency (count) of the i$^{th}$ ΔT row and j$^{th}$ ΔP column cells. Then,

$$Row\ mean = Mean(dt) = \frac{\sum_{i=1}^{r} m_i B_i}{\sum_{i=1}^{r} mi} \quad \ldots\ldots\ldots\ (3)$$

where $B_i$ is the $i^{th}$ row $\Delta T$ midpoint value (r=row). The term "$i^{th}$ $\Delta T$ row and $j^{th}$ $\Delta P$ column cell" may also need an explanation. If a horizontal line is drawn through the midpoint in row $i$, and a vertical line through the midpoint in column $j$, the two lines will cross each other in a cell. This cell has the coordinates "$i^{th}$ row and $j^{th}$ column cell ". As an example, the data of Table I are used to calculate the mean row value. Application of the equations (2) and (3) gives a row mean with respect to columns equal to 0.135 seconds (14.9/110.25). The calculations are shown in more detail in Table II.

Table II. Computation of row (latency) mean with respect to columns (amplitude).

| mi | $\Delta Ti$ | mi x $\Delta Ti$ |
|---|---|---|
| 3x1.25+12x1.75=24.75 | 0.125 | 3.0925 |
| 16x1.25+12x1.75+8x2.25=59 | 0.135 | 7.965 |
| 7x1.25+5x1.75+4x2.25=26.5 | 0.145 | 3.8425 |
| Sum=110.25 | | 14.9 |
| **Row mean: 14.9/110.25 = 0.135 seconds** | | |

Second, the $\Delta P$ mean value (columns), with respect to the $\Delta T$ value (rows), is determined. The column $\Delta P$ mean value are found using the same approach as used for finding the mean row $\Delta T$ value. First, the $m_j$ for each $\Delta P$ column is found, as given in equation (4).

$$m_j = \sum_{i=1}^{i=r} B_i w_{ij} \ldots\ldots\ldots\ldots\ (4)$$

where $B_i$ is the $i^{th}$ row $\Delta T$ midpoint, and referring to a $\Delta T$ group value and $w_{ij}$ is the frequency for the $i^{th}$ row and $j^{th}$ column. Then,

$$Column\ mean = Mean(dP) = \frac{\sum_{j=1}^{j=c} m_j A_j}{\sum_{j=1}^{j=c} mi} \quad \ldots\ldots\ldots\ (5)$$

where $A_j$ is the $j^{th}$ column $\Delta P$ value midpoint (c=column). The calculations are shown in Table III, using the equations (4) and (5), the column mean with respect to rows will be equal to 1.64 mmHg (14.9/9.055).

Table III. Computation of column (amplitude) mean with respect to rows (latency).

| Column | mj | $\Delta Pj$ | mj x $\Delta Pj$ |
|---|---|---|---|
| 1 | | | |
| 2 | 3x0.125+16x0.135+7x0.145=3.55 | 1.25 | 4.4375 |
| 3 | 12x 0.125 + 12 x 12x0.135 + 5 x 0.145 = 3.845 | 1.75 | 6.72875 |
| 4 | 8x0.135+4x0.145+4x=1.66 | 2.25 | 3.735 |
| 5 | | | |
| Sum | | 9.055 | 14.9 |

(continued)

| Column | mj | ΔPj | mj x ΔPj |
|---|---|---|---|
| Column mean: 14.9/9.055 = 1.64 mmHg | | | |

Finally, it should be mentioned that balanced position may as well be determined within a one-dimensional matrix (termed second matrix). Such a matrix is used for determining balanced position of occurrences of rise-time coefficients during a given time sequence. It may be used for other one-dimensional matrix variables/relations as well. In this situation the rise time coefficients are plotted in a one-dimensional matrix of pre-defined rise time coefficients. In such a one-dimensional frequency distribution we have two variables $x_i$, and $w_i$ ($x_i$ equal to the value of each observation, $w_i$ equal to the frequency or count). $x_i$ is comparable to ΔP/ΔT and $w_i$ is comparable to the number of occurrences of the various ΔP/ΔT combinations. The mean of this distribution may be computed according to equation 6:

$$\overline{X} = \frac{\sum_{i=1}^{k} x_i w_i}{\sum_{i=1}^{k} w_i}, k = number\ of\ observations\ ........(6)$$

It has been discussed computation of a two-dimensional matrix of combinations of amplitude (ΔP) and latency (ΔT) combinations (referred to as the first matrix), and also computation of a one-dimensional matrix of combinations of rise-time coefficients (ΔP/ΔT) (referred to as the second matrix). It should be noted that these are examples, and not intended to limit the scope of the invention. A matrix may contain any of the single pressure wave parameters discussed in this document, and any combinations are possible. Matrixes may be computed for any type of pressure. The numbers of groups may be selected.

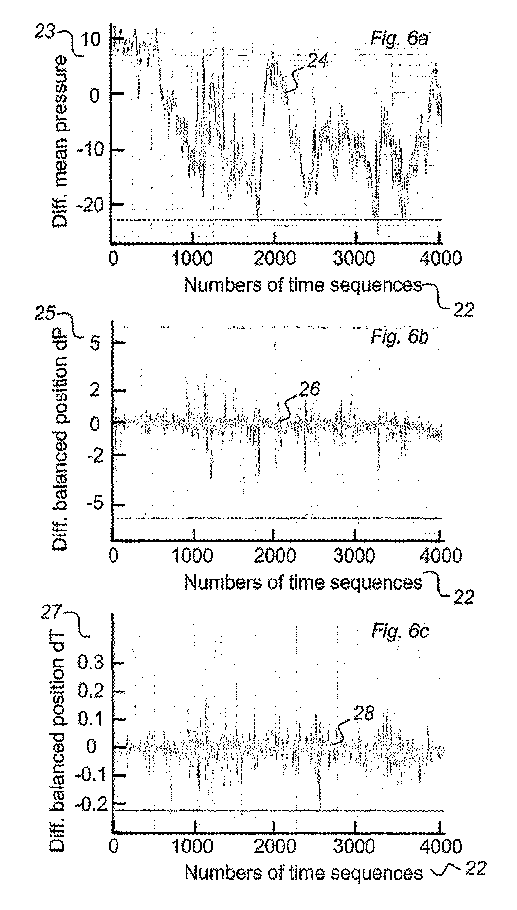

Reference is now given to FIGS. **6a-c.** This figure illustrates differences between computation of absolute mean pressure and single pressure wave parameters. Intracranial pressure was measured simultaneously by means of two different sensors located within the brain parenchyma, separated by 1-2 centimetres. Intracranial pressure would be expected to be similar, given the narrow location between the sensors. Intracranial pressure was measured simultaneously by the two sensors. Since measurements had identical time reference, the single pressure wave parameters computed within given time sequences of 6 seconds duration could be compared time sequence for time sequence. For each time sequence 11 absolute mean pressure was computed according to Metode 2, as well as balanced position of amplitude (ΔP) and balanced position of latency (ΔT). Time sequence for time sequence in the continuous series of time sequences, differences between the two pressure recordings were computed with regard to absolute mean pressure (FIG. **6a**), balanced position of amplitude (ΔP) (FIG. **6b**), and balanced position of latency (FIG. **6c**). With reference to FIG. **6a,** on the x axis is shown the number of the time sequences **22.** In this recording period, the first time sequence starts with number 1 and the last time sequence ends with number 4100, indicating the start and end of the recording period, respectively. Thus, the continuous recording period consisted of a series of 4100 continuous time sequences **11,** corresponding to 24600 seconds (6.8 hours). On the x axis is indicated the scale for differences in absolute mean pressure **23.** The differential pressure curve **24** shows the trend distribution of differences in absolute pressure within identical time sequences for two pressure recordings with different sensors. Each plot within said differential plot **24** presents differences within identical time sequences for absolute mean pressure, wherein differences related to pressures measured by either of said two sensors. A large variation in absolute pressures is shown. For time sequence number 1000, absolute mean pressure was 20.2 mmHg for sensor 1 and 33.3 mmHg for sensor 2, with a difference in mean pressure of -13.1 mmHg. In FIG. **6b** is indicated on the y axis differences in balanced position of amplitude (ΔP) **25** and the series of time sequences **22** on the x axis. The differential curve of balanced position **26** shows for consecutive time sequences differences in balanced position of amplitude (ΔP). The trend curve show **25** that differences in balanced position of amplitude (ΔP) between identical time sequences are minimal. For time sequence number 1000, balanced position of amplitude was 5.2 mmHg for sensor 1 and 5.6 mmHg for sensor 2. Difference in balanced position was 0.4 mmHg. In the same way, differences in balanced position of latency are shown in Fig. **6c.** In FIG. **6c** is shown on the y axis differences in balanced position of latency (ΔT) 27 and on the x axis the consecutive series of time sequences **22.** The differential trend plot of balanced position of latency (ΔT) **28** show minimal differences between balanced position of latency (AT) for different pressure curves using different sensors with identical time sequences. The results presented in FIG. **6a-c** suggest that pressures are less reliably predicted by absolute mean pressure, as compared to balanced position of amplitude (ΔP) and latency (ΔP). Again it should be noted that differences were computed on a beat to beat basis since the time reference was identical.

With regard to time sequences, absolute mean pressure and balanced position of either amplitude (ΔP) or latency (ΔP) are only few of many different single pressure wave parameters. The various parameters related to single pressure waves **1** are discussed in the following. An inventive step of this invention is to store in a database the single pressure wave related parameters computed according to the invention. The single wave 1 related parameters relate to said time sequences **11**. Before creating a database, the duration of said time sequences may be selected. The inventor suggest that the duration preferably should be of 5-15 seconds duration. Specific durations of said time sequences are not a limitation. The inventor computed a database wherein time sequences of 6 seconds duration were selected. Within each time sequence the single wave related parameters may either be related to each of the individual single pressure waves within said time sequence, or to the group of individual single waves within said time sequence.

For each of the individual single pressure waves **1** within said time sequence **11,** the following parameters are stored (referred to as Single wave parameters 1-6):

1. Absolute pressure value for diastolic minimum ($P_{min}$) **2** value for each individual single pressure wave **1** (i.e. accepted $P_{min}/P_{max}$ pair) during said time sequence **11**.
2. Absolute pressure value for systolic maximum ($P_{max}$) **3** value for each individual single pressure wave **1** (i.e. accepted $P_{min}/P_{max}$ pair) during said time sequence **11**.
3. Absolute mean pressure for each accepted single wave 1 (i.e. accepted $P_{min}/P_{max}$ pair) (related to each accepted $P_{min}/P_{max}$ pair), that is mean pressure from $P_{min}$ to $P_{min}$ (wavelength) **7** for each individual single pressure wave **1** during said time sequence **11**.
4. Relative amplitude (ΔP) **4** pressure value for each individual single pressure wave **1** (i.e. accepted $P_{min}/P_{max}$ pair) during said time sequence **11**.
5. Relative latency (AT) **5** value for each individual single pressure wave **1** (i.e. accepted $P_{min}/P_{max}$ pair) during said time sequence **11**.
6. Relative rise time coefficient (ΔP/ΔT) **6** value for each individual single pressure wave **1** (i.e. accepted $P_{min}/P_{max}$ pair) during said time sequence **11**.

For the group of single pressure waves within said time sequence 11, the following parameters are stored (referred to as time sequence parameters 1-12):

1. Numbers of single waves ($N_{SW}$) during said time sequence.
2. Single pressure wave derived heart rate **16,** computed as numbers of single pressure waves **1** divided with the total duration of wavelengths ($P_{min}$ to $P_{min}$) **7** of single pressure waves within said time sequence 11.
3. Single pressure wave derived heart rate **16,** computed as numbers of single pressure waves **1** divided with the duration of said time sequence **11** wherein said single pressure waves occur.
4. Absolute mean pressure for said time sequence **11,** computed as the sum of absolute mean pressure (entire wavelength **7** from $P_{min}$ to $P_{min}$) for all individual single waves 1 during said time sequence **11,** divided by numbers of single waves within said time sequence **11** (referred to as Method 2).
5. Standard deviation for mean pressure of mean pressure for the individual single waves **1** occurring during said time sequence **11**.
6. Standard deviation for diastolic minimum ($P_{min}$) **2** during said time sequence, which is computed as standard deviation for diastolic minimum ($P_{min}$) **2** of all individual single waves **1** during said time sequence **11**.
7. Standard deviation for systolic maximum ($P_{max}$) **3** during said time sequence **11,** which is computed as standard deviation for systolic maximum ($P_{max}$) **3** of all individual single waves **1** occurring during said time sequence **11**.
8. Standard deviation for amplitude (ΔP) **4** of all individual single pressure waves **1** occurring during said time sequence **11**.
9. Standard deviation for latency (ΔT) **5** of all individual single pressure waves **1** occurring during said time sequence **11**.
10. Standard deviation for rise time coefficient (ΔP/ΔT) **6** of all individual single pressure waves **1** occurring during said time sequence **11**.
11. Balanced position of amplitude (ΔP)/latency (ΔT) combinations in said amplitude/latency matrix (referred to as first matrix).
12. Balanced position of rise time coefficients (ΔP/ΔT) in rise time coefficient matrix (referred to as second matrix).

All the single pressure wave related parameters are computed for each time sequence. Given that the duration of each time sequence is set to 6 seconds, an individual recording of 10 hours consists of 6000 time sequences. For example, the sole parameter *Balanced position of amplitude (ΔP) and latency (ΔT)* consists of two values (e.g. 0.12 seconds|6.25 mmHg), that gives 12000 values during a 10 hours recording period (20 values/minute x 60 minutes/hr x 10 hrs). These 12000 values include 6000 values of balanced position amplitude (ΔP) **4** and 6000 values of balanced position latency (ΔT) **5**. Thus, for every time sequence the single wave related parameters are stored. The inventor first created a database based on time sequences of 6 seconds. At an early stage the database consisted of several millions of said time sequences. Since the data are continuous, it is easy to change the duration of the time sequence (e.g. to 5 seconds duration), though the computer requires time to process the digital data.

The database serves several purposes; an important purpose is to determine relationships between the different single wave parameters. Since relationships between several parameters within identical time sequences may be determined, it is also possible to determine one parameter as a function of two or more other parameters. For example, for one individual pressure recording, the single pressure wave parameters within each time sequence may be related. This procedure may be computed in a scatter plot with one parameter on the y axis and the other on the x axis. An example is given. A continuous pressure recording of 10 hours contains a total of 6000 time sequences, each lasting 6 seconds. Provided that 5400 of 6000 time sequences are accepted from said pressure recording, this pressure recording contains a total of 5400 values of balanced position of amplitude ($\Delta$P) **4** and 5400 values of latency (AT) **5.** In a scatter plot each value refers to a combination of balanced position of amplitude ($\Delta$P) **4** and balanced position of latency ($\triangle$T) 5. The relationships between the 5400 plots may further be determined by computing the best fitted curve. Goodness of fit may be determined by various strategies. For a given relationship, it is the experience of the inventor that the goodness of fit as well as the spread of the plot may differ among different pressure recordings.

The relationships between parameters may as well be determined for a group of individual pressure recordings. For example, for a group of 100 individual pressure recordings the relationships between balanced positions of amplitude ($\Delta$P) **4** and latency ($\Delta$T) **5** may be determined. Given that each individual pressure recording contains an average of 5400 values of balanced position of amplitude ($\Delta$P) **4** and 5400 values of balanced position of latency ($\Delta$T) **5,** an averaged total of 540 000 values of each variable is available. Various mathematical procedures are possible to determine the relationships between these variables in such a large sample. A scatter of 540 000 plots may be made. A relationship may as well be made by a random selection of the total material. The invention does not limit to a particular strategy for determining relationships within a large material, as various mathematical strategies are possible.

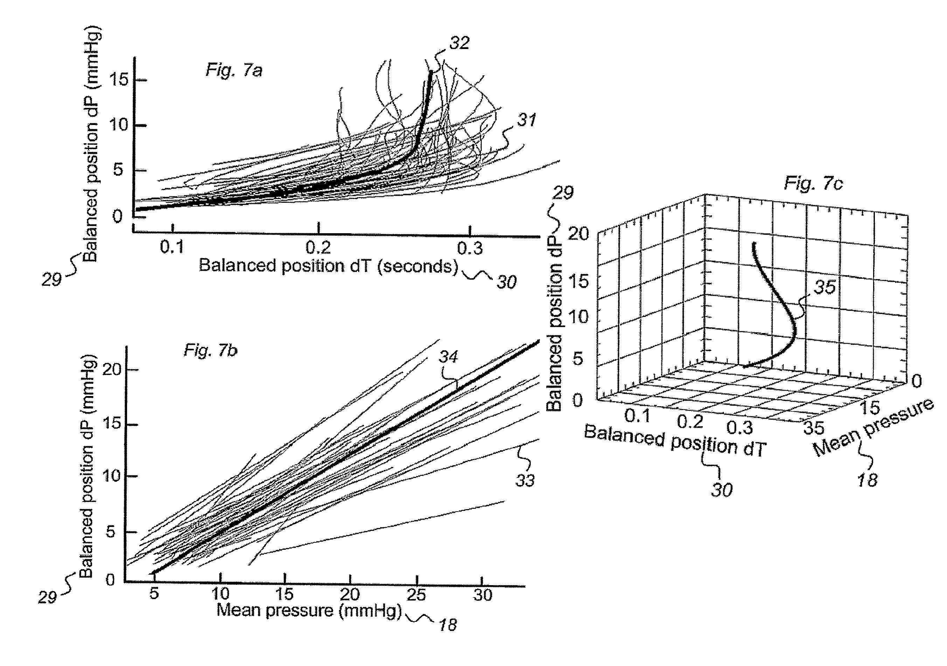

In FIGS. **7a-c** addresses the topic of determining relationships between single pressure wave parameters within a group of individual pressure recordings. This is an example of one strategy, though this example is not intended to limit the scope of the invention. The data presented in FIGS. **6a-c** represents a total of 40 individual pressure recordings. These 40 individual pressure recordings contain a total of 330540 individual time sequences **11,** each lasting 6 seconds. First, each individual of said 40 individual pressure recordings were considered. For each individual pressure recording, the best fitted equation is determined for ranges of the parameters wherein the curve is based. For example, for one pressure recording the best fitted equation was applicable for the amplitude ($\Delta$P) ranges of 2.5 to 6.7 mmHg, whereas another pressure recording determined the best fitted equation for the amplitude ($\Delta$P) ranges of 5.4 to 12.0 mmHg. Second, the best fitted curves **31** from the individual pressure recordings were sampled within a scatter of all 40 individual pressure recordings. In FIG. **7a** the y axis shows the balanced position values of amplitude ($\Delta$P) values **29,** and the x axis the balanced position of latency ($\Delta$T) **30.** In FIG. **6b** is balanced position of amplitude ($\Delta$P) values **29** on the y axis plotted against absolute mean pressure (computed according to Method 2) **18** on the x axis. In FIG. **6a** is indicated the regression line **31** corresponding to individual pressure recordings of the relationship between balanced position of amplitude ($\Delta$P) and latency ($\Delta$T). The total regression line **32** for all individual pressure recordings is shown. This corresponds to the relationship between balanced position of amplitude ($\Delta$P) and latency ($\Delta$T) for the whole group of 40 individual pressure recordings including 330540 time sequences. The relationship is exponential. In FIG. **7b** is shown the regression lines **33** for the individual pressure recordings for the relationship between balanced position of amplitude ($\Delta$P) 29 and absolute mean pressure **18.** The total regression line **34** for all individual regression lines concerning relationship between balanced position of amplitude ($\Delta$P) **29** and absolute mean pressure **18** is shown in FIG. **6b.** The equation of the total regression line **32** of FIG. **6a** may be combined with the equation of the total regression line 34 of FIG. **6b.** Since both equations contain the variable balanced position of amplitude ($\Delta$P) **29,** it is possible to compute one of the variables as a function of the others. This aspect is further illustrated in FIG. **6c,** wherein the variables balanced position of amplitude ($\Delta$P) **29,** balanced position of latency ($\triangle$T) **30,** and absolute mean pressure **18** are plotted are plotted in a 3D graph. In FIG. **7c** is shown a graphical presentation of the three-dimensional regression line **35** based on the equation of the three variables balanced position of amplitude ($\Delta$P) **29,** balanced position of latency ($\Delta$T) **30,** and absolute mean pressure **18.** The equation of this three-dimensional regression line shows one variable as a function of the two other variables. Independent of the method, the following model was computed for this particular relationship: Mean pressure = a + $b_1$ x $\Delta$P + $b_2$ x $\Delta$T$^3$. On this basis an equation was determined:

$$\text{Predicted mean pressure} = 3.214 + 1.3 \text{ x } \Delta P + 63.609 \text{ x } \Delta T^3 \dots\dots (7)$$

It should be noted that this equation is relevant for the data presented in FIGS. **7a** and **7b.** For other materials, other equations may be computed. This equation is included to give an example of how one single pressure wave parameter may be expressed as a function of two other single pressure wave parameters. The data have been selectively chosen, but contains a huge number of comparisons. The data shown in these figures consist of 330 540 individual time sequences **11,** each lasting 6 seconds. Nevertheless, an important question is how to establish a database of individual pressure recordings that may provide a reliable relationship between the single pressure wave parameters. According to this invention, selected criteria are established to determine whether or not an individual pressure recording may be included in determining the relationship between single pressure wave parameters. Not all scatter plots of single pressure wave parameters are useful for determining fitted curve formulas since the variation within the plot may be very large. Determination of goodness of fit for regression lines of individual pressure recordings may be made by various strategies. An important issue is also to determine which parameters that do or do not influence on each other.

An important aspect of determining relationships between single pressure wave related parameters is an inventive procedure of giving weights to cells within a matrix. Reference has been made to said first matrix of amplitude ($\Delta$P) and latency ($\triangle$T) combinations (see Table I). The cells within the matrix described in Table I may be represented as weight values. Instead of the word *weight,* the word *score* might be used. In this description the word weight is preferred. A weighted cell value means that each cell in said matrix (see Table I) is represented by one value instead of two values corresponding to the respective column and row numbers. According to the invention, weight values are made on the basis of observations. In this context, observations refer to the relationships established by means of the database.

Reference is now given to Table IV that is a weight matrix. The group names, ranges and midpoints correspond to the amplitude/latency matrix shown in Table I. For example, the amplitude ($\Delta$P) group named 1.5 mmHg includes amplitude values equal to or larger than 1.5 mmHg, but less than 2.0 mmHg, with group midpoint value equal to 1.75 mmHg. The latency ($\Delta$T) group termed 0.11 seconds includes latency values equal to or larger than 0.11 seconds, but less than 0.12 seconds, with group midpoint value of 0.115 seconds. With reference to Table IV, the equation of the relationships presented in FIG. **7c** was used to give each individual cell in said matrix a weight value. The weight value was considered as equal to predicted mean (Predicted mean pressure = 3.214 + 1.3 x $\Delta$P + 63.609 x $\Delta$T$^3$). The equation was applied to each amplitude and latency group within said matrix. The equation describes the predicted mean value as a function of the balanced position of amplitude ($\Delta$P) and latency ($\Delta$T) values. With reference to Table IV, the group midpoint values of amplitude ($\Delta$P) and latency ($\Delta$T) groups were used as input values to the equation to give each cell a predicted mean value. For example, by using the equation 7 related to FIG. **7c** (Predicted mean pressure = 3.214 + 1.3 x $\Delta$P + 63.609 x $\Delta$T$^3$), the cell corresponding to amplitude ($\Delta$P) group 1.5 mmHg (with group midpoint 1.75 mmHg) and latency ($\Delta$T) group 0.11 seconds (with group midpoint 0.115 seconds) would be represented with the predicted mean pressure value of 5.59 mmHg. In this example the whole matrix is weighted according to the equation computed according to the relationships presented in FIGS. **7a** to **7c.** Based on the relationships presented in FIGS. **7a** to **7c,** it is also possible to compute one individual equation for each amplitude ($\Delta$P) group within said matrix. Thereby, each equation is applicable for ranges of amplitude ($\Delta$P), for example the ranges 0.5<$\Delta$P≤1.0 mmHg.

It should be noted that the numbers and equations presented are used as illustrative examples and are not intended to limit the scope of the invention. The weight values computed depend on the relationships determined according to the observational data. Which absolute pressure levels that correspond to which balanced position $\Delta$P and $\Delta$T levels depend on the fitted curve equations computed for the particular data set. The invention sets no limitations concerning which types of observations the relationships are based on. Preferentially the plots should be based on a group of patients. However, separate plots may be made for different patient groups, patient ages, and disease states. These curves may to some extent differ depending on the types of pressures measured, compartments where pressures are measured, method by which pressures are measured, age of patient in whom pressures are measured, as well as disease state of the patient. In these situations certain weight matrixes may be used only for particular patient groups or disease states.

Table TV. A part of a weight matrix wherein the number in each matrix cell is a weight value that is a function of the amplitude (dP) and latency (dT) values.

| Group name | Group range | | 0.5 | 1 | 1.5 | 2 | 2.5 | 3 | 3.5 | 4 | 4.5 | 5 |
|---|---|---|---|---|---|---|---|---|---|---|---|---|
| | | | 0.5<dP<1.0 | 1.0<dP<1.5 | 1.5<dP<2.0 | 2.0<dP<2.5 | 2.5<dP<3.0 | 3.0<dP<3.5 | 3.5<dP<4.0 | 4.0<dP<4.5 | 4.5<dP<5.0 | 5.0<dP<5.5 |
| | | Group midpoint | 0.75 | 1.25 | 1.75 | 2.25 | 2.75 | 3.25 | 3.75 | 4.25 | 4.75 | 5.25 |
| 0.1 | 0.10<dT<0.11 | 0.105 | 4.26 | 4.91 | 5.56 | 6.21 | 6.86 | 7.51 | 8.16 | 8.81 | 9.46 | 10.11 |
| 0.11 | 0.11<dT<0.12 | 0.115 | 4.29 | 4.94 | 5.59 | 6.24 | 6.89 | 7.54 | 8.19 | 8.84 | 9.49 | 10.14 |
| 0.12 | 0.12<dT<0.13 | 0.125 | 4.31 | 4.96 | 5.61 | 6.26 | 6.91 | 7.56 | 8.21 | 8.86 | 9.51 | 10.16 |
| 0.13 | 0.13<dT<0.14 | 0.135 | 4.35 | 5.00 | 5.65 | 6.30 | 6.95 | 7.60 | 8.25 | 8.90 | 9.55 | 10.20 |
| 0.14 | 0.14<dT<0.15 | 0.145 | 4.38 | 5.03 | 5.68 | 6.33 | 6.98 | 7.63 | 8.28 | 8.93 | 9.58 | 10.23 |
| 0.15 | 0.15<dT<0.16 | 0.155 | 4.43 | 5.08 | 5.73 | 6.38 | 7.03 | 7.68 | 8.33 | 8.98 | 9.63 | 10.28 |
| 0.16 | 0.16<dT<0.17 | 0.165 | 4.47 | 5.12 | 5.77 | 6.42 | 7.07 | 7.72 | 8.37 | 9.02 | 9.67 | 10.32 |
| 0.17 | 0.17<dT<0.18 | 0.175 | 4.53 | 5.18 | 5.83 | 6.48 | 7.13 | 7.78 | 8.43 | 9.08 | 9.73 | 10.38 |
| 0.18 | 0.18<dT<0.19 | 0.185 | 4.59 | 5.24 | 5.89 | 6.54 | 7.19 | 7.84 | 8.49 | 9.14 | 9.79 | 10.44 |
| 0.19 | 0.19<dT<0.20 | 0.195 | 4.66 | 5.31 | 5.96 | 6.61 | 7.26 | 7.91 | 8.56 | 9.21 | 9.86 | 10.51 |
| 0.2 | 0.20<dT<0.21 | 0.205 | 4.74 | 5.39 | 6.04 | 6.69 | 7.34 | 7.99 | 8.64 | 9.29 | 9.94 | 10.59 |
| 0.21 | 0.21<dT<0.22 | 0.215 | 4.82 | 5.47 | 6.12 | 6.77 | 7.42 | 8.07 | 8.72 | 9.37 | 10.02 | 10.67 |
| 0.22 | 0.22<dT<0.23 | 0.225 | 4.91 | 5.56 | 6.21 | 6.86 | 7.51 | 8.16 | 8.81 | 9.46 | 10.11 | 10.76 |
| 0.23 | 0.23<dT<0.24 | 0.235 | 5.01 | 5.66 | 6.31 | 6.96 | 7.61 | 8.26 | 8.91 | 9.56 | 10.21 | 10.86 |
| 0.24 | 0.24<dT<0.25 | 0.245 | 5.12 | 5.77 | 6.42 | 7.07 | 7.72 | 8.37 | 9.02 | 9.67 | 10.32 | 10.97 |
| 0.25 | 0.25<dT<0.26 | 0.255 | 5.24 | 5.89 | 6.54 | 7.19 | 7.84 | 8.49 | 9.14 | 9.79 | 10.44 | 11.09 |
| 0.26 | 0.26<dT<0.27 | 0.265 | 5.37 | 6.02 | 6.67 | 7.32 | 7.97 | 8.62 | 9.27 | 9.92 | 10.57 | 11.22 |
| 0.27 | 0.27<dT<0.28 | 0.275 | 5.51 | 6.16 | 6.81 | 7.46 | 8.11 | 8.76 | 9.41 | 10.06 | 10.71 | 11.36 |
| 0.28 | 0.28<dT<0.29 | 0.285 | 5.66 | 6.31 | 6.96 | 7.61 | 8.26 | 8.91 | 9.56 | 10.21 | 10.86 | 11.51 |
| 0.29 | 0.29<dT<0.30 | 0.295 | 5.82 | 6.47 | 7.12 | 7.77 | 8.42 | 9.07 | 9.72 | 10.37 | 11.02 | 11.67 |
| 0.3 | 0.30<dT<0.31 | 0.305 | 5.99 | 6.64 | 7.29 | 7.94 | 8.59 | 9.24 | 9.89 | 10.54 | 11.19 | 11.84 |
| 0.31 | 0.31<dT<0.32 | 0.315 | 6.18 | 6.83 | 7.48 | 8.13 | 8.78 | 9.43 | 10.08 | 10.73 | 11.38 | 12.03 |
| 0.32 | 0.32<dT<0.33 | 0.325 | 6.37 | 7.02 | 7.67 | 8.32 | 8.97 | 9.62 | 10.27 | 10.92 | 11.57 | 12.22 |

EP 1 770 545 A2

| Group name |  |  | 0.5 | 1 | 1.5 | 2 | 2.5 | 3 | 3.5 | 4 | 4.5 | 5 |
|---|---|---|---|---|---|---|---|---|---|---|---|---|
|  | Group range |  | 0.5<dP<1.0 | 1.0<dP<1.5 | 1.5<dP<2.0 | 2.0<dP<2.5 | 2.5<dP<3.0 | 3.0<dP<3.5 | 3.5<dP<4.0 | 4.0<dP<4.5 | 4.5<dP<5.0 | 5.0<dP<5.5 |
| 0.33 | 0.33<dT<0.34 | 0.335 | 6.58 | 7.23 | 7.88 | 8.53 | 9.18 | 9.83 | 10.48 | 11.13 | 11.78 | 12.43 |
| 0.34 | 0.34<dT<0.35 | 0.345 | 6.80 | 7.45 | 8.10 | 8.75 | 9.40 | 10.05 | 10.70 | 11.35 | 12.00 | 12.65 |
| 0.35 | 0.35<dT<0.36 | 0.355 | 7.03 | 7.68 | 8.33 | 8.98 | 9.63 | 10.28 | 10.93 | 11.58 | 12.23 | 12.88 |
| 0.36 | 0.36<dT<0.37 | 0.365 | 7.28 | 7.93 | 8.58 | 9.23 | 9.88 | 10.53 | 11.18 | 11.83 | 12.48 | 13.13 |
| 0.37 | 0.37<dT<0.38 | 0.375 | 7.54 | 8.19 | 8.84 | 9.49 | 10.14 | 10.79 | 11.44 | 12.09 | 12.74 | 13.39 |
| 0.38 | 0.38<dT<0.39 | 0.385 | 7.82 | 8.47 | 9.12 | 9.77 | 10.42 | 11.07 | 11.72 | 12.37 | 13.02 | 13.67 |
| 0.39 | 0.39<dT<0.40 | 0.395 | 8.11 | 8.76 | 9.41 | 10.06 | 10.71 | 11.36 | 12.01 | 12.66 | 13.31 | 13.96 |
| 0.4 | 0.40<dT<0.41 | 0.405 | 8.41 | 9.06 | 9.71 | 10.36 | 11.01 | 11.66 | 12.31 | 12.96 | 13.61 | 14.26 |

In FIG. **8** is illustrated how determination of single wave distribution may be used in the real-time and online monitoring of pressures. The first sequence of events is indicated in FIGS. **8a** to **8c,** providing a schematic overview of computing balanced position of amplitude/latency combinations within consecutive time sequences **11**. Pressure signals from any type of pressure sensor may be sampled, and the single waves **1** identified. Within each time sequence **11** (illustrated with a duration of 5 seconds; Fig. **8a**), the single pressure waves **1** are identified. In FIG. **8a** is indicated seven single pressure waves within the time sequence **11** of 5 seconds. For all accepted single pressure waves 1 within said time sequence **11,** the single pressure wave parameters amplitude ($\Delta P$) **4** and latency ($\Delta T$) **5** are plotted in a first matrix **36**. The first matrix **36** in FIG. **8b** represents only a small part of a matrix **36** for intracranial pressure. The numbers **37** presented in the matrix are numbers of single waves with different combinations of latency **5** and amplitude **4** during said time sequence **11.** An alternative to presenting numbers is presentation of percentages of combinations. With reference to FIG. **8b,** the numbers of occurrences of single waves with amplitude of 2.0 mmHg and a latency of 0.13 seconds was 2 during said time sequence of 5 seconds. For this matrix **36** the numerical value **38** of balanced position of amplitude and latency combinations was 0.12 seconds|2.4 mmHg (FIG. **8c**). This combination of 0.12|2.4 refers to the single pressure wave combination wherein latency **5** was 0.12 seconds and amplitude 4 2.4 mmHg. In fact, various terms may be used concerning balanced position such as centre of mass or centroid. In this context, the balanced position refers to the mean frequency distribution of amplitude **4** and latency **5** combinations within said time sequence **11** and represented in said matrix **36,** as previously described in detail. The procedure in FIG. **8a-c** is repeated every new time sequence **11** in the continuous series of time sequences **11** during a continuous pressure monitoring. Accordingly a new balanced position is computed each new 5 seconds in this particular example. During real-time monitoring these numerical value combinations may be updated each 5 seconds. The numerical value combinations **38** may be presented on the display of an apparatus or a monitor.

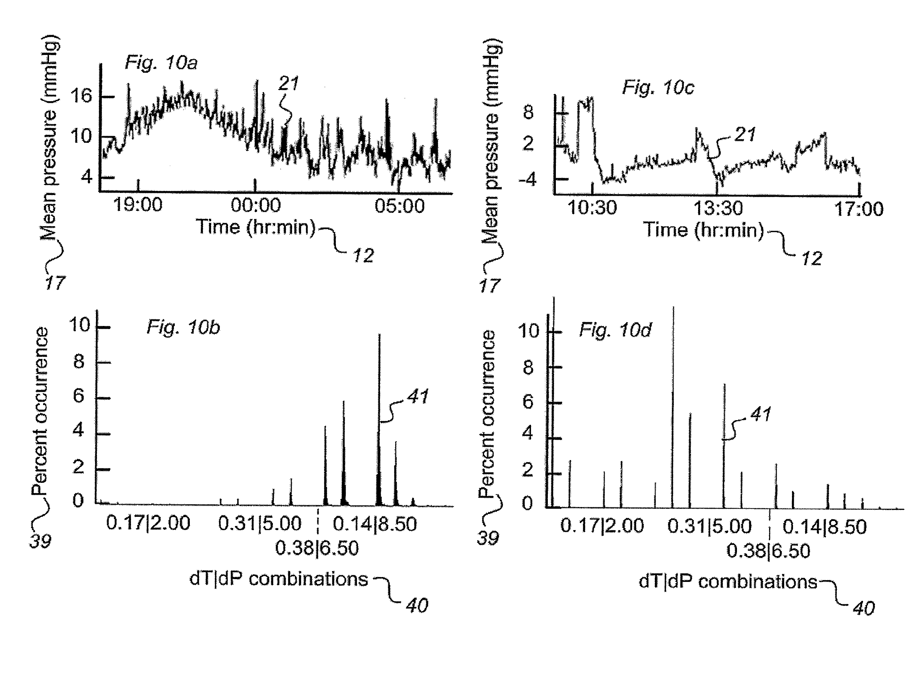

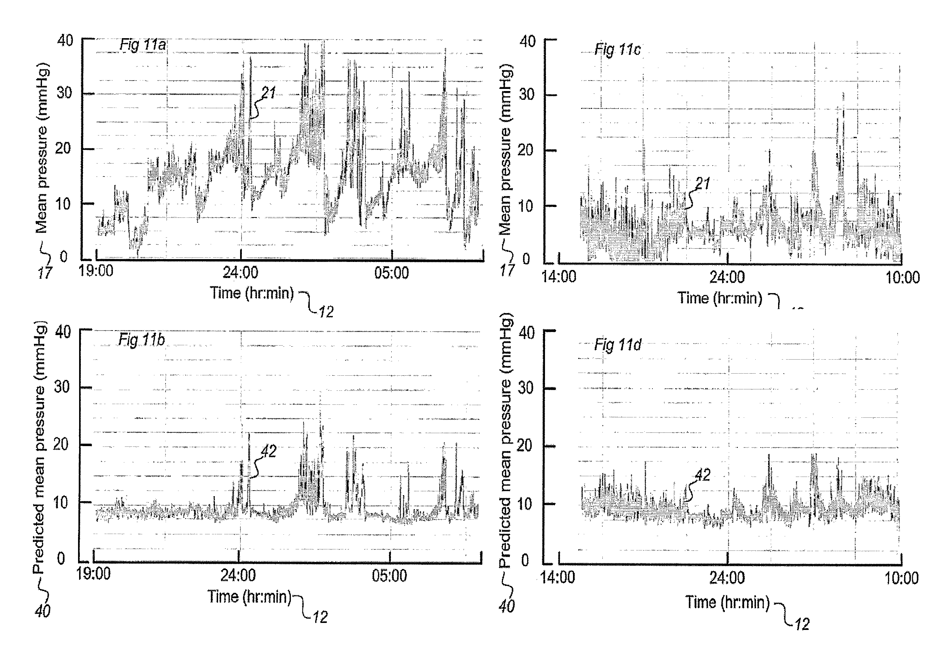

During on-line monitoring it may be difficult for the physician or nurse to relate to new numerical values presented each 5 seconds. Therefore, various examples of presentations are given in FIG. **8d-f.** In all these examples the two-dimensional values of balanced position are presented as one-dimension values. According to this invention this is made possible by weighting of the matrix cells. Thereby, the two-dimensional balanced position may be represented by a one-dimensional weighted value. In FIG. **8d** is shown a histogram presentation with numbers or proportions **39** on the y axis and weighted balanced position **40** values on the x axis. For example, such a histogram may reveal for a given recording period the total distribution of weighted values of balanced positions **40** of amplitude and latency within the time sequences occurring during said recording period, as further shown in FIGS. **9b, 9d, 10b,** and **10d.** In such a histogram each bar **41** represents a given weighted value of balanced position of amplitude and latency within said 5-second time sequences, with the numbers or proportions of said weighted values represented on the x axis. In FIG. **8e** is presented weighted values of balanced position **40** of amplitude/latency combinations in a trend plot, with time scale **12** on the x axis and weighted values of balanced position **40** on the y axis. In the trend plot **42** each plot represents a weighted value of balanced position **40** of amplitude and latency within a time sequence of 5 seconds. Thus, the trend plot shows the output of analysis of each time sequence in a continuous series of time sequences. Criteria may be selected for handling excluded time sequences. Examples of trend plots **42** of weighted values are further given in FIGS. **11b** and **11d.** A third alternative of presenting single wave distribution is indicated in FIG. **8f.** A modification of a so-called pressure volume curve may be computed for a large number of individuals. Such a curve may depend on age. The inventor has computed so-called pressure volume curves for adults by means of so-called infusion tests, previously described in US patent application 09/843,702 and

International Patent Application PCT/NO 02/00164. On the X-axis is indicated change of volume **43.** On the Y-axis is indicated the balanced position **40** of amplitude and latency. During so-called infusion tests a fixed volume is applied to the intracranial compartment, for example with similar volume changes each 5 seconds. During each 5 seconds the single waves are monitored with computation of matrix 36. The balanced position **40** may be computed, and expressed on the Y-axis. The fixed volume change **43** is indicated on the X-axis. The inventor has been able to compute such pressure volume curves for a large number of patients. Thereby reference curves have been computed, indicating both normal and abnormal curves. Such reference curves may be shown on the display of the apparatus or on other monitor systems. During real-time monitoring the balanced position of single wave combinations may be plotted in relation to the modified pressure volume curve, for example each 5 seconds. Thereby, real-time and online update of balanced position for a single case may be computed real-time and online and related to a reference curve. Thereby information about compliance/elastance is obtained.

Reference is now given to FIG. **9** that shows two different intracranial pressure recordings in one single case. Pressures were recorded simultaneously (with identical time reference) by means of one sensor placed within the brain parenchyma (FIGS. **9a-9b**) and one sensor placed epidurally (FIGS. 9c-9d). An epidural placement means that the sensor is placed outside the dura mater actually mimicking non-invasive pressure monitoring since the sensor is not placed within the cavity in which pressure is measured. Both pressures measured within brain parenchyma and epidurally are relative to atmospheric pressure, and represent absolute pressures. For both pressure curves (FIGS. **9a** and **9c**) are presented the absolute pressures **17** on the y axis and the time scale **12** on the x axis. Both the x axes show identical time sequences, making a beat to beat comparison possible. It should be noted that the absolute pressures differ for the pressure curve **21** for parenchyma (FIG. **9a**) and epidural (FIG. **9c**) pressures. For FIG. **9a** mean intracranial pressure for the whole recording period was 5.9 mmHg. For FIG. **9c** mean intracranial pressure for the whole recording period was 8.35 mmHg. In this context, the absolute mean pressure values of 5.9 and 8.35 mmHg actually represent the mean of all 5 second time intervals during the total recording period. On the other hand, the distribution of single waves was nearly identical between parenchyma and epidural measurements, as indicated in the histogram located to the right for each pressure curve (FIGS. **9b** and **9d**). Whereas the pressure curves **21** show absolute pressures the histograms refer to single waves defined by relative pressures. The amplitudes of the single waves are computed as relative pressure differences. In FIGS. **9b** and **9d** is shown the histogram wherein all weighted values **40** of balanced positions of amplitude and latency during said recording period is shown on the x axis. On the y axis the percentage occurrence **39** is indicated, which is how often a single wave with a certain combination of latency/amplitude occurs in percentage of the total number of single waves during the recording period. Each balanced position of amplitude/latency combinations represented by a weight value is represented by one bar **41** in the histogram. For example, the label on the X-axis of 0.38|6.50 refers to single waves with a combination of latency of 0.38 seconds and amplitude of 6.50 mmHg. In this example the values 0.31|5.00, 0.38|6.50 and 0.14|8.50 refer to balanced position values. These values might as well be referred to as index values when a weighted matrix is used. The histograms presented in FIG. **9b** and **9d** actually show weighted balanced position values 40 of all time sequences **11** during the recording period illustrated in FIGS. **9a** and **9c.** The bar corresponding to the latency|amplitude combination of 0.38|6.50 indicates the percentage by which single wave occurred as related to the total numbers of single waves. For parenchyma (Fig. **9b**) and epidural (Fig. **9d**) pressures, the single wave distribution is nearly identical. These pressure recordings illustrate several important aspects of the invention: Absolute pressures recorded by the conventional strategy and illustrated in the pressure curves give no reliable description of the pressures. Pressures within the brain parenchyma and the epidural space as revealed by the pressure curves were markedly different. Both the absolute pressures and the morphology of the curve were different. Continuous pressure recordings are most accurately described by the single wave distribution. The histogram presentations of the single wave distribution were nearly identical for pressure recordings within the brain parenchyma and the epidural space. Therefore, single wave distribution may be equally presented whether or not the sensor is placed within the cavity pressure is measured. Results such as these gave the idea to compute single wave distribution in infants by monitoring fontanel pressure non-invasively by applying a sensor on the fontanel. When the results presented in FIGS. **9a-d** are presented in a differential plot as described in FIGS. **6a-c,** absolute mean pressure is compared time sequence for time sequence. Also balanced position of amplitude and latency is compared time sequence for time sequence, since both pressure curves have identical time reference. Such a differential plot of the results presented in FIGS. **9a-d** showed a marked difference in absolute mean pressures between parenchymatous and epidural measurements. The differences in balanced positions of amplitude and latency between time sequences with identical reference were minimal.

Reference is now given to FIGS. **10a-d** illustrating how matrix and histogram presentations change before and after intervention in one single case. With reference to the first recording period, is presented the pressure curve **21** (FIG. **10a**) and histogram (FIG. **10b**). The trend plot **21** (FIG. **10c**) and histogram (FIG. **10d**) for the second recording period also is presented. The matrix **36** corresponding to FIGS. **10a** and **10b** is shown in Table V, and the matrix **36** corresponding to FIGS. **10c** and **10d** in Table VI. An explanation of the different amplitude **4** and latency **5** groups presented in Tables V and VI are further given for Table 1. The numbers **37** within cells of matrix **36** presented in Table I represent absolute numbers, whereas the numbers in Tables V and VI refer to percentages. Before intervention, the combination of amplitude **4** of 7.5 mmHg and latency **5** of 0.26 seconds occurred in 0.17% of the total numbers of single waves. After intervention, the combination of amplitude **4** of 7.5 mmHg and latency **5** of 0.26 seconds did not occur. In this particular example, the matrixes **36** including amplitudes **4** and latencies **5,** were standardized to a recording period and a heart rate. Non-standardized numbers may as well be presented. The standardized recording period was set to one hour. The actual heart rate was variable during the recording period, but was standardized to a standardized heart rate of 70 beats a minute. With reference to histograms (FIG. **10c, 10d**), on the y axis is shown percentage of occurrence **39** that is how often a single wave with a certain latency|amplitude combination occurred in percentage of the total number of single waves. On the x axis is shown the different weighted latency|amplitude combinations **40.** As an example; in these histograms the label 0.14|8.50 on the x axis refers to single waves with latency **5** of 0.14 seconds and amplitude **4** of 8.50 mmHg. Accordingly, the bar **41** corresponding to the label 0.14|8.50 shows the percentage of single waves with this combination occurring as percentage of total number of single waves during a standardized recording time of one hour and a standardized heart rate of 70 beats a minute. Before (FIGS. **10a, 10b**) and after (FIGS. **10c, 10d**) intervention, the matrixes (Tables V and VI) and histograms showed a marked difference in single wave distribution, with a change of the single wave distribution in a more normal direction.

FIG. **10** also may serve as an example of distribution of balanced positions of amplitude and latency combinations **39** for a whole recording period, wherein the total distribution is presented in matrixes in Tables V and VI. Thereby, balanced positions of amplitude/latency combinations within individual time sequences for the total recording period are presented as numbers in proportion **38** of the total number. In this situation, the bars **41** shown in the histogram refer to balanced positions of amplitude/latency combinations during selected time sequences **11.** The histograms in FIGS. **10b** and **10d** illustrate how balanced position of amplitude and latency changes from one pressure recording to another. With reference to the matrix in Table V, balanced position of latency/amplitude combinations of 0.24 seconds|4 mmHg occurred in 5.02% in the matrix **36** presented in Table V, whereas this combination did not occur in matrix of Table VI. In this context, it should be noted that the matrixes **36** usually are computed each time sequence **11** with determination of balanced position of amplitude/latency combinations for each individual time sequence **11** in a series of continuous time sequences. The matrixes **36** presented in Tables V and VI, on the other hand, show the distribution of balanced positions of amplitude and latency combinations for the whole recording period.

Table V. Matrix of amplitude (dP) and latency (dT) combinations corresponding to continuous pressure recordings presented in FIGS. **10a** and **10b**.

| dT\dP | 1 | 1.5 | 2 | 2.5 | 3 | 3.5 | 4 | 4.5 | 5 | 5.5 | 6 | 6.5 | 7 | 7.5 | 8 | 8.5 | 9 | 9.5 | 10 | 10.5 | 11 |
|---|---|---|---|---|---|---|---|---|---|---|---|---|---|---|---|---|---|---|---|---|---|
| **0.1** | | 0.02 | 0.05 | 0.21 | 0.13 | | | | | | | | | | | | | | | | |
| **0.11** | | | | 0.09 | 0.05 | | | | | | | | | | | | | | | | |
| **0.12** | | | | | | | | | | | | | | | | | | | | | |
| **0.13** | | | 0.01 | 0.02 | 0.04 | | | | | | | | | | | | | | | | |
| **0.14** | | | 0.01 | | | | | | | | | | | | | | | | | | |
| **0.15** | | | | | | | | | | | | | | | | | | | | | |
| **0.16** | | | | | | | | | | | | | | | | | | | | | |
| **0.17** | | 0.03 | 0.34 | 0.05 | 0.02 | | | | | | | | | | | | | | | | |
| **0.18** | | 0.01 | 0.33 | 0.19 | 0.04 | | | | | | | | | | | | | | | | |
| **0.19** | | | | | | | | | | | | | | | | | | | | | |
| **0.2** | | 0.01 | 0.26 | 0.65 | 0.9 | 0.11 | | | | | | | | | | | | | | | |
| **0.21** | | | 0.14 | 0.71 | 1.51 | 0.58 | 0.21 | 0.12 | 0.02 | | | | | | | | | | | | |
| **0.22** | | | | | | | | | | | | | | | | | | | | | |
| **0.23** | | | 0.41 | 1.55 | 4.5 | 3.93 | 2.59 | 1.01 | 0.39 | 0.17 | 0.04 | 0.01 | 0.04 | 0.03 | | 0.03 | | | | | |
| **0.24** | | | 0.37 | 1.93 | 3.83 | 5.95 | 5.02 | 3.32 | 1.92 | 1.13 | 0.5 | 0.29 | 0.23 | 0.13 | 0.18 | 0.13 | 0.06 | 0.01 | | | |
| **0.25** | | | | | | | | | | | | | | | | | | | | | |
| **0.26** | | | 0.18 | 1.78 | 4.22 | 7.59 | 9.75 | 7.05 | 3.36 | 1.95 | 1.03 | 0.52 | 0.23 | 0.17 | 0.11 | 0.07 | 0.06 | 0.09 | 0.05 | 0.03 | 0.02 |
| **0.27** | | | 0.03 | 0.35 | 1.08 | 2.32 | 3.66 | 2.81 | 1.36 | 0.88 | 0.32 | 02 | 0.1 | 0.05 | 0.01 | | 0.02 | 0.01 | 0.01 | | |
| **0.28** | | | 0.04 | 0.03 | 0.06 | 0.26 | 0.4 | 0.47 | 0.3 | 0.2 | 0.06 | 0.03 | 0.04 | 0.01 | | | | | | | |
| **0.29** | | | 0.01 | 0.01 | | | | 0.01 | 0.01 | | | | | | | | | | | | |
| **0.3** | | | | 0.01 | | | | | | | | | | | | | | | | | |
| **0.31** | | | | | | | | | | | | | | | | | | | | | |
| **0.32** | | | | 0.01 | 0.01 | | | | | | | | | | | | | | | | |
| **0.33** | | | | | | | | | | | | | | | | | | | | | |
| **0.34** | | | | 0.01 | | | | | | | | | | | | | | | | | |

Table VI. Matrix of amplitude (dP) and latency (dT) combinations corresponding to continuous pressure recordings presented in FIGS. **10c** and **10d.**

| dT\dP | 1 | 1.5 | 2 | 2.5 | 3 | 3.5 | 4 | 4.5 | 5 | 5.5 | 6 | 6.5 | 7 | 7.5 | 8 | 8.5 | 9 | 9.5 | 10 | 10.5 | 11 |
|---|---|---|---|---|---|---|---|---|---|---|---|---|---|---|---|---|---|---|---|---|---|
| **0.1** | | 10.5 | 14.6 | 0.63 | | | | | | | | | | | | | | | | | |
| **0.11** | 0.02 | 2.04 | 2.75 | 0.07 | | | | | | | | | | | | | | | | | |
| **0.12** | | | | | | | | | | | | | | | | | | | | | |
| **0.13** | | 1.17 | 2.08 | 0.11 | | | | | | | | | | | | | | | | | |
| **0.14** | 0.01 | 1.17 | 2.72 | 0.23 | | | | | | | | | | | | | | | | | |
| **0.15** | | | | | | | | | | | | | | | | | | | | | |
| **0.16** | | 0.73 | 1.5 | 0.05 | | | | | | | | | | | | | | | | | |
| **0.17** | 0.02 | 6.74 | 11.8 | 0.3 | 0.03 | | | | | | | | | | | | | | | | |
| **0.18** | | 5.29 | 5.54 | 0.31 | | | | | | | | | | | | | | | | | |
| **0.19** | | | | | | | | | | | | | | | | | | | | | |
| **0.2** | 0.01 | 3.64 | 7.26 | 0.63 | 0.04 | | | | | | | | | | | | | | | | |
| **0.21** | | 0.82 | 2.17 | 0.34 | 0.03 | 0.01 | | | | | | | | | | | | | | | |
| **0.22** | | | | | | | | | | | | | | | | | | | | | |
| **0.23** | | 0.79 | 2.6 | 1.38 | 0.13 | 0.05 | | | | | | | | | | | | | | | |
| **0.24** | | 0.21 | 1 | 0.91 | 0.12 | 0.01 | | | | | | | | | | | | | | | |
| **0.25** | | | | | | | | | | | | | | | | | | | | | |
| **0.26** | | 0.11 | 0.73 | 1.42 | 0.36 | 0.06 | | | | | | | | | | | | | | | |
| **0.27** | | 0.04 | 0.54 | 0.91 | 0.28 | 0.07 | | | | | | | | 0.01 | | | | | | | |
| **0.28** | | | 0.34 | 0.61 | 0.39 | 0.05 | 0.01 | | 0.01 | | | | | | | | | | | | |
| **0.29** | | | 0.07 | 0.12 | 0.12 | 0.04 | | | 0.01 | | | | | | | | | | | | |
| **0.3** | | | 0.07 | 0.06 | 0.04 | 0.01 | | | | | | | | | | | | | | | |
| **0.31** | | | | | | | | | | | | | | | | | | | | | |
| **0.32** | | | 0.02 | 0.09 | 0.05 | 0.04 | | | | | | | | | | | | | | | |
| **0.33** | | | 0.02 | 0.05 | 0.1 | 0.03 | | | | | | | | | | | | | | | |
| **0.34** | | | | 0.07 | 0.18 | | | | | | | | | | | | | | | | |

EP 1 770 545 A2

(continued)

| dT\dP | 1 | 1.5 | 2 | 2.5 | 3 | 3.5 | 4 | 4.5 | 5 | 5.5 | 6 | 6.5 | 7 | 7.5 | 8 | 8.5 | 9 | 9.5 | 10 | 10.5 | 11 |
|---|---|---|---|---|---|---|---|---|---|---|---|---|---|---|---|---|---|---|---|---|---|
| 0.35 | | | | 0.02 | 0.13 | | | | | | | | | | | | | | | | |
| 0.36 | | | | 0.02 | 0.12 | | | | | | | | | | | | | | | | |
| 0.37 | | | | | 0.02 | | | | | | | | | | | | | | | | |

Reference is now given to FIG. **11**. Two continuous intracranial pressure curves **21** are shown in FIGS. **11a** and **11c**. On the y axis is the scale of absolute mean pressure **17** computed according to conventional technology (Method 1). The time scale **12** is on the x axis. The trend curve **21** presents a continuous plot of mean pressures wherein mean pressure is computed each time sequence (6 seconds duration in this case) in a continuous series of time sequences. Mean pressures **21** are much higher in FIG. **11a** than in FIG. **11c**. Below the absolute pressure curves **21** (FIGS. **11a** and **11c**) is shown the corresponding trend plot **42** of balanced position of amplitude and latency computed each 6 seconds. The two-dimensional balanced position has been computed as a one-dimensional value, as described for Table IV. By applying the equation (no. 7) used to weight the matrix **36** presented in Table IV, each balanced position of amplitude **4** and latency **5** was expressed as a weight value. The weighted value is given the term *predicted mean pressure* **40**, shown on the y axis of FIGS. **11b** and **11d**. The time scales **12** are identical for FIGS. **11a** and **11b,** and for **11c** and **11d**. Thus, the trend plots **42** of weighted balanced positions (*predicted mean pressure*) show balanced position of amplitude **4** and latency **5** computed each time sequence **11** (6 seconds) that is expressed as a weighted values termed predicted mean pressure **40**. Each plot in said trend plot **42** represents the balanced position each time sequence 11 in a continuous series of time sequences. The trend plots **42** of weighted balanced positions are similar for FIGS. **11b** and **11d**. Thus, though absolute mean pressure curves **21** differed markedly, weighted trend plots **42** of balanced position did not differ. The trend plots **42** of weighted balanced position in FIGS. **11b** and **11d** correspond to the trend plot **42** schematically presented in FIG. **8e**. The histograms presented in FIGS. **9c, 9d, 10b,** and **10d** showing weighted balanced position **40** on the x axis correspond to the histogram presentation shown in FIG. **8d**. In both the trend plots and histograms weighted values **40** of balanced position of amplitude 4 and latency **5** are presented. These are examples of presentations and are not intended to limit the scope of the invention.

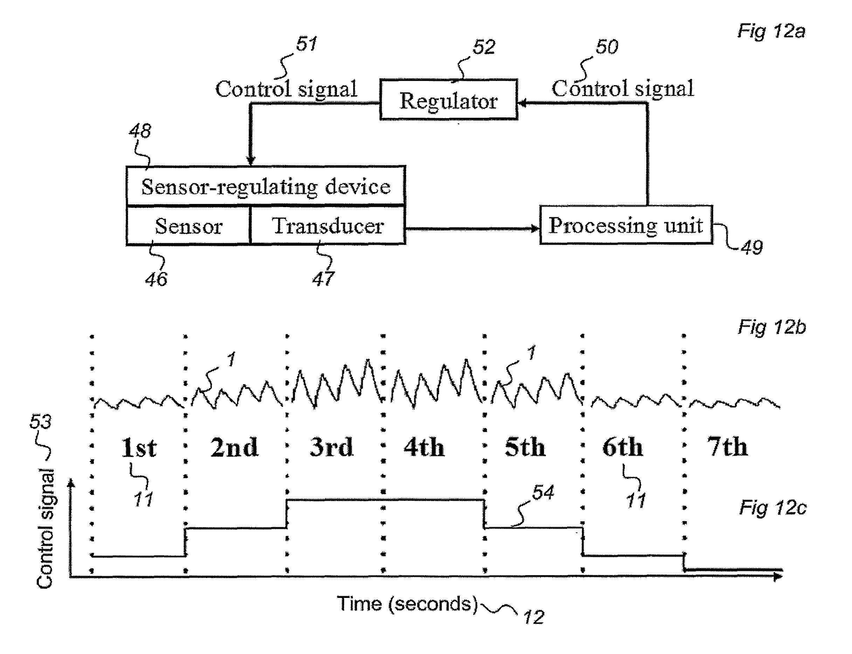

Reference is now given to FIG. **12**. The present invention uses the method for analysis of single pressure waves **1** to provide a strategy for more optimal single pressure wave detection, particularly when non-invasive devices are used for pressure monitoring. A schematic representation is provided in FIG. **12a**. The invention may be used in conjunction with various types of sensors **46** providing signals indicative of pressure. The sensor device itself is not a part of the invention. It is well known from the prior art that sensors **46** operate together with pressure transducers **47**. An excitation signal is applied from the transducer 47 to the sensor **46,** and the sensor **46** gives back a new signal indicative of the pressure to the transducer **47**. The transducer **47** then processes the signal to another signal that is more suitable for further signal processing. The sensor device may as well incorporate a sensor-regulating device **48** that regulates how the sensor **46** is applied to the object wherein pressure is measured. In general terms, the sensor device consists of a sensor **46**, transducer **47** and sensor-regulating device **48**. The pressure signals from the pressure transducer **47** are further converted into pressure-related digital data with a time reference, and analysed according to the invention within the processing unit **49**. The method for analysis of single pressure waves provides an output that gives a first control signal **50** to a regulator device **52**. Said regulator device may be a transducer that converts the first control signal **50** from the processing unit **49** into another second control signal **51**. The second control signal **51** produced by the regulator **52** modifies the performance of the sensor-regulating device **48**. The sensor-regulating device modifies the mode by which the sensor/transducer is able to sample signals indicative of pressure. Thereby, the inventive method for analysis of single pressure waves is used to control and modify the sampling mode of signals derivable from a pressure sensor device.

Though the sensor device itself is not a part of the invention, nor a method by which such a sensor device is used on an animal or human body cavity, some examples are given to illustrate the concept, though this represents no limitation of the scope of the invention. First, applanation tonometry are widely used for non-invasive pressure measurement. Pressure gradients exist across the walls of a pressurised elastic sphere. When a pressure sensor is applied to the surface of the flattened area, no pressure gradient exists over the flattened portion. Pressure measurements can be made when a constant pressure is applied to the flattened area. Applanation tonometry may for example be used in non-invasive blood pressure monitoring, monitoring of ocular pressure (i.e. pressure within the ocular bulb), and even monitoring fontanel pressure in infants with an open fontanel. The pressure sensor **46** consists of the pressure element that is in contact with the skin or eye bulb. Signals from the sensor **46** are converted within the pressure transducer **47**. When pressures are measured using the principles of applanation tonometry, it is well known that the pressure pulsation's detected by the tonometer depend on the pressure by which the tonometer is applied to the measurement surface. With increasing pressure from the tonometer, pressure waves increase until the waves with highest amplitudes are recorded. The pressure by which the applanation tonometry is applied to the surface determines quality of signal detection. Therefore, devices for applanation tonometry may include a sensor-regulating device **48,** which controls the pressure by which the tonometer is applied to the surface. Such a sensor-regulating device **48** may be an inflatable balloon housed in a solid frame, under control of a pneumatic system. Such a sensor-regulating device **48** provides the opportunity for controlled inflation of air into an air chamber of the sensor. The pneumatic system is automatic and controlled by a processing unit **49,** in the way that the air chamber pressure is automatically regulated to show the best single pressure waves. Other sensors **46** apply Doppler signals to detect pressure related signals. The signal detected by the Doppler may be modified within the transducer **47**. In such a system the sensor-regulating device **48** incorporates a system wherein Doppler signals are applied to/receive from the object, including acquisitions of direction of signal emission as well as the signal quantity and quality. The emission and detection of Doppler signals heavily depend on the angulations of the signal emitting source. In this situation the sensor-regulating device **48** determines how Doppler signal are applied to the object, including acquisitions of signal direction and strength. When the sensor **46** and transducer **47** use acoustic signals the sensor-regulating device **48** may as well control signal direction and signal quantity and quality. Since the sensor device itself is not a particular feature of the invention, a more detailed description is not given.

The procedure for controlling and changing the sampling mode of signals derivable from a pressure sensor device is further illustrated in FIGS. **12b** and **12c**. The digital signals are sampled and analysed during short time sequences **11** (e.g. of 3 seconds duration) in a continuous series of short time sequences **11**. Said analysis of single wave **1** related parameters within said time sequences **11** is performed by a processing unit **49**. For each time sequence **11** a number of single pressure wave **1** related parameters are computed, as already described in more detail. One or more of the following parameters are included:

(1) absolute mean pressure for each identified single pressure wave **1** [wavelength **7** ($P_{min}$ - $P_{min}$)] within said time sequence **11**;

(2) mean of mean pressure for all identified single pressure waves **1** [wavelength **7** ($P_{min}$ - $P_{min}$)] within said time sequence **11**;

(3) standard deviation of absolute mean pressure for all identified single pressure waves **1** [wavelength **7** ($P_{min}$ - $P_{min}$)] within said time sequence **11**;

(4) numbers of single pressure waves **1** during said time sequence **11**;

(5) single pressure wave derived heart rate **16** during said time sequence **11**;

(6) relative pressure amplitude ($\Delta P$) **4** value for each identified single pressure wave **1** [wavelength 7 ($P_{min}$ - $P_{min}$)] within said time sequence **11**;

(7) standard deviation of relative pressure amplitude ($\Delta P$) **4** values for all identified single pressure waves **1** [wavelength 7 ($P_{min}$ - $P_{min}$)] within said time sequence **11**;

(8) relative latency ($\triangle T$) **5** value for each identified single pressure wave **1** [wavelength **7** ($P_{min}$ - $P_{min}$)] within said time sequence **11**;

(9) standard deviation of relative latency (AT) **5** values for all identified single pressure waves **1** [wavelength **7** ($P_{min}$ - $P_{min}$)] within said time sequence **11**;

(10) rise time ($\Delta P/\Delta T$) coefficient **6** for each identified single pressure wave **1** [wavelength **7** ($P_{min}$ - $P_{min}$)] within said time sequence **11**;

(11) standard deviation of rise time ($\Delta P/\Delta T$) coefficient **6** for all identified single pressure waves **1** [wavelength **7** ($P_{min}$- $P_{min}$)] within said time sequence **11**;

(12) relative latency (AT) **5** value for each identified single pressure wave **1** [wavelength **7** ($P_{min}$ - $P_{min}$)] within said time sequence **11**;

(13) balanced position within said first matrix **36** for combinations of single pressure wave amplitude ($\Delta P$) **4** and latency ($\Delta T$) **5** values within said time sequence **11**; and

(14) balanced position within said second matrix **36** for combinations of single pressure wave rise-time ($\Delta P/\Delta T$) **6** coefficients within said time sequence **11.**

FIG. **12b** illustrates seven time sequences **11** (1st, 2nd, 3rd, 4th, 5th, 6th, and 7th) where single pressure wave 1 detection is modified. Single pressure wave 1 detection is most optimal during the 3rd and 4th time sequences wherein the amplitudes **4** are most evident. For each time sequence **11** said analysis procedure is applied to all single pressure waves **1.** Selected criteria are applied to the analysis output, and would reveal most optimal single wave detection for the 3rd and 4th time sequences 11 in this example.

The output of said analysis within the processing unit **49** establishes or amends a first control signal **50** that is applied to a regulator **52**. The mode of the first control signal **50** is determined by the output of said single pressure wave related analysis. Between each new time sequence, a first control signal **50** may be determined, depending on criteria applied to the analysis results. The first control signal **50** is converted within a regulator **52**. The regulator **52** may be considered as a transducer converting the first control signal **50** into another second control signal **51**. The regulator **52** deliverable second control signal **51** may depend on the type of sensor-regulating device **48** incorporated in the sensor device. Therefore, the deliverable second control signal **51** may modify a sensor-regulating device **48** in a wide sense. An example is given with reference to applanation tonometry wherein a pneumatic system controls the pressure by which the sensor **46** is applied to the surface. The second control signal **51** delivered from the regulator **52** may determine the pressure level within the pneumatic system, which determines the pressure by which the tonometer is applied to the surface. This example is further illustrated in FIG. **12c.** In this situation, the control signal level **53** determines the pressure level within a pneumatic system. The pressure level **54** is increased for each new of the first three time sequence **11** (1$^{st}$, 2$^{nd}$, and 3$^{rd}$ time sequence). As indicated in FIG. **12b**, single pressure wave 1 detection is improved for each new of said time sequences **11.** The control signal level **53** and accordingly the pressure level **54** is kept constant between the 3$^{rd}$ and 4$^{th}$ time sequences **11** (FIG. **12c**) wherein single waves **1** remain more or less unchanged. The control signal level **53** and accompanying pressure level **54** is reduced during the subsequent 5$^{th}$, 6$^{th}$, and 7$^{th}$ time sequences **11,** wherein also single wave **1** detection is reduced. The second control signal **51** produced by the regulator **52** may as well control a sensor regulating device **48** using Doppler signals. In this situation the second control signal **51** may control the angulations and signal quality and quantity of the sensor-regulating device **48** using Doppler signals. Furthermore, when a sensor device utilizes acoustic signals, the second control signal **51** may control the sensor regulating device **48** determining the quality and quantity of acoustic signals applied to the surface wherein pressures are measured.

The results of said single pressure wave **1** analysis are compared for different time sequences **11.** Changes in a first control signal **50** and subsequent in another second control signal **51** both produce modifications of the sensor-regulating device **48**. The deliverable control signals corresponding to analysis output wherein single pressure wave parameters meet one or more selectable criteria would be used during subsequent pressure monitoring. The selectable criteria correspond to the control signal wherein the most optimum single pressure wave detection is obtained.

It is not within the scope of the invention to limit the strategy by which the process is performed. The system provides feedback interaction between the processing unit **49** performing said analysis, the deliverable first control signal **50** to the regulator **52** controlling and changing the deliverable second control signal **51** applied to the sensor-regulating device **48**. It is described one example of the interactive operation of the regulator **52** and processing unit **49** related to the description shown in FIGS. **12b** and **12c**, though this represents no limitation of the scope of the invention. During a given time period of for example 30 seconds, the regulator **52** delivers a total of ten different second control signals **51,** each separated by 3 seconds. During the period of 30 minutes, ten modifications of the sensor-regulating device **48** are made between each of said 3 second intervals. During the ten time sequences **11** (each lasting 3 seconds), single pressure waves are analyzed within the processing unit **49.** For each of said time sequences **11** the output of the analysis within the processing unit **49** determines a first control signal **50** that corresponds to the second control signal **51** from the regulator **52.** Accordingly, each time sequence **11** corresponds to a second control signal **51** from said regulator **52** that corresponds to an output of single pressure wave **1** analysis within said processing unit **49** that further corresponds to a first control signal **50** to the regulator **52.** These corresponding values are determined for each individual of the ten time sequences 11 during said recording period of 30 minutes. During the subsequent pressure monitoring, the processing unit **49** provides the first control signal **50** that corresponds to optimum single pressure wave 1 detection. This first control signal **50** from the processing unit **49** to the regulator **52** gives another second control signal **51** to the sensor-regulating device **48,** further enabling the sensor-regulating device **48** for optimum single pressure wave 1 detection. The procedure of determining the most optimum control signal (here exemplified as lasting 30 minutes) may be reiterated at selected intervals during an ongoing pressure measurement. Various modifications of this process are possible. For example, during an ongoing pressure monitoring the processing unit **49** may automatically determine the second control signal **51** from the regulator **48,** wherein single pressure wave 1 detection is most optimum.

**Claims**

1.  A method for analyzing pressure signals derivable from pressure measurements on or in a body of a human being or animal, comprising the steps of sampling said signals at specific intervals, and converting the pressure signals into pressure-related digital data with a time reference,
    wherein for selectable time sequences the method comprises the further steps of:

    a) identifying from said digital data the single pressure waves in said pressure signals,
    b) computing single pressure wave related parameters of said single pressure waves, selected from such as:

- absolute mean pressure,
- amplitude,
- latency, and
- rise time coefficient,

c) identifying numbers of single pressure waves with pre-selected parameter values of such waves with respect to said parameters such as absolute mean pressure, amplitude, latency and rise time coefficient,

d) plotting the numbers of occurrences of single pressure waves with pre-selected values of amplitude and latency in a first matrix, determining balanced position of amplitude and latency combinations in said first matrix, and presenting the balanced positions obtained as numerical values or as related to weight values, and/or

e) plotting the numbers of occurrences of single pressure waves with pre-selected values of rise time coefficients in a second matrix, determining balanced positions of rise time coefficients in said second matrix, and presenting the balanced positions obtained as numerical values or as related to weight values.

2. A method according to claim 1, wherein said method is applied to continuous pressure-signals during said selectable time sequences.

3. A method according to claim 2, wherein said selectable time sequences lies in the range 5-15 seconds.

4. A method according to claim 2 or 3, wherein single pressure waves occurring between two time sequences are included in one or the other of said two time sequences according to pre-selected criteria.

5. A method according to claim 2, wherein a continuous series of said selected time sequences constitutes a continuous pressure recording period.

6. A method according to claim 5, wherein any of said selected time sequences are accepted or rejected for further analysis according to selected criteria.

7. A method according to claim 1, comprising the further steps of applying the method to all continuous pressure-signals for each of said time sequences in a continuous series of said time sequences during a continuous measurement period.

8. A method according to claim 1, wherein said identifying step includes determination of all minimum (valleys) and maximum (peak) pressure values in said signal.

9. A method according to claim 1, wherein said identifying step of single pressure waves relates to identifying a minimum pressure value ($P_{min}$) related to a diastolic minimum value and a maximum pressure value ($P_{max}$) related to a systolic maximum value of said single pressure wave.

10. A method according to claim 1, wherein said identifying step of single pressure waves includes determination of a minimum-maximum ($P_{min}/P_{max}$) pair of said single pressure wave.

11. A method according to claim 1, wherein said identifying step includes determining at least one of the single pressure wave parameters related to correct minimum-maximum pressure ($P_{min}/P_{max}$) pairs, said parameters selected from the group of: amplitude ($\Delta P$), latency ($\Delta T$), and rise time coefficient ($\Delta P/\Delta T$).

12. A method according to claim 1, wherein said single pressure wave amplitude relates to pressure amplitude = $\Delta P$ = systolic maximum value ($P_{max}$) - diastolic minimum value ($P_{min}$).

13. A method according to claim 1, wherein said single pressure wave latency relates to time latency = $\Delta T$ = time sequence wherein pressures increases from diastolic minimum pressure ($P_{min}$) to systolic maximum pressure ($P_{max}$).

14. A method according to claim 1, wherein said single pressure rise time coefficient relates to the relationship $\Delta P/\Delta T$ between amplitude $\Delta P$ and latency $\Delta T$.

15. A method according to claim 1, wherein said identifying step includes exclusion of minimum-maximum pressure ($P_{min}/P_{max}$) pairs with either amplitude ($\Delta P$), latency ($\Delta T$) or rise time coefficient ($\Delta P/\Delta T$) values outside pre-selected thresholds.

**16.** A method according to claim 1, wherein said single pressure wave parameters elected from the group of: amplitude ($\Delta P$), latency ($\Delta T$) and rise time coefficients ($\Delta P/\Delta T$) are relative values only and independent of any zero pressure level.

**17.** A method according to claim 9, wherein said systolic maximum pressure value ($P_{max}$) is one of three peak values occurring in said single pressure wave.

**18.** A method according to claim 17, wherein

- a first (P1) of said three peak values in said single pressure wave has an amplitude related to the top of the percussion wave,
- a second (P2) of said three peak values has an amplitude related to a tidal wave portion of said single pressure wave, and
- a third (P3) of said three peak values has an amplitude related a dichrotic wave portion of said single pressure wave.

**19.** A method according to claim 17 or 18 further comprising the step of calculating one or more rise time coefficients $\Delta P/\Delta T$ based on a ratio between said amplitude and latency values.

**20.** A method according to claim 1, wherein absolute mean pressure for each individual of said single pressure waves relates to mean pressure during the time of the pressure waveform, i.e. from diastolic minimum pressure ($P_{min}$) to diastolic minimum pressure ($P_{min}$).

**21.** A method according to claim 20, wherein mean pressure for an individual single pressure wave is the sum of pressure levels within said pressure wave divided by numbers of pressure samples.

**22.** A method according to claim 20, wherein mean pressure for an individual single pressure wave is the area under a curve (AUC) for said single pressure wave.

**23.** A method according to claim 1, wherein absolute mean pressure for said selected selectable time sequences is the sum of absolute mean pressure (wavelength $P_{min}$ -$P_{min}$) for all individual single pressure waves during said time sequence divided by the numbers of single pressure waves within said identical time sequence.

**24.** A method according to claim 1, wherein absolute mean pressure of single pressure waves relates to absolute pressure relative to atmospheric pressure.

**25.** A method according to claim 1, wherein single pressure waves are rejected when absolute pressure values of single pressure wave diastolic minimum pressure ($P_{min}$) and systolic maximum pressure ($P_{max}$) of said single waves are outside selected threshold values.

**26.** A method according to claim 1, wherein heart rate during said time sequence is equal to numbers of single pressure waves during said time sequence divided by the duration of said time sequence.

**27.** A method according to claim 1, wherein heart rate during said time sequence is equal to numbers of single pressure waves during said time sequence divided by the sum of wavelengths ($P_{min}$ - $P_{min}$) for all of said individual single pressure waves during said time sequence.

**28.** A method according to claim 1, wherein a time sequence of pressure recordings is accepted or rejected according to single pressure wave related parameters within said time sequence.

**29.** A method according to claim 28, wherein said time sequence is of a duration in the range 5-15 seconds.

**30.** A method according to claim 28, wherein a time sequence is rejected when standard deviation of absolute pressures of minimum/maximum ($P_{min}/P_{max}$) pair values of said single pressure waves is outside selected threshold values.

**31.** A method according to claim 28, wherein a time sequence is rejected when standard deviation of one or more of single pressure wave parameters selected from the group of: amplitude ($\Delta P$), latency ($\Delta T$) and rise time coefficient ($\Delta P/\Delta T$) is outside selected threshold values.

32. A method according to claim 28, wherein a time sequence is rejected when the number of single pressure waves within said time sequence is outside a selected threshold value.

33. A method according to claim 28, wherein a time sequence is rejected when single pressure wave derived heart rate for said time sequence is outside a selected threshold value.

34. A method according to claim 28, wherein a time sequence is rejected when the number of single pressure waves for said time sequence deviates outside selected values, as compared to the number of single pressure waves derived from another pressure recorded during identical time sequence with identical time reference.

35. A method according to claim 28, wherein a time sequence is rejected when single pressure wave derived heart rate for said time sequence deviates outside selected values, as compared to single pressure wave derived heart rate from another pressure recorded during identical time sequence with identical time reference.

36. A method according to claim 28, wherein a time sequence is rejected when single pressure wave derived heart rate for said time sequence deviates outside selected values, as compared to heart rate derived from other source.

37. A method according to claim 36, wherein said other source is pulse oxymetry or electrocardiography.

38. A method according to anyone of claims 28 -37, wherein said rejection or acceptance of time sequences is performed repeatedly during ongoing pressure measurements.

39. A method according to anyone of claims 28 - 37, wherein a log is made for accepted and rejected time sequences during a recording period.

40. A method according to claim 1, comprising the further step of creating a matrix based on determination of a number of single pressure waves with pre-selected values related to one or more single pressure wave related parameters, and indicating for each matrix cell at respective intersections in said first and/or second matrix the number of occurrence of matches between specific parameters of said single pressure waves.

41. A method according to claim 40, wherein a matrix is created based on determining numbers of single pressure waves with pre-selected values related to amplitude ($\Delta P$) and latency ($\Delta T$), wherein one axis of the first matrix is related to an array of pre-selected values of pressure amplitude ($\Delta P$), wherein the other axis in said first matrix is related to an array of pre-selected latencies ($\Delta T$), and wherein indicating for each matrix cell at respective intersections in said matrix a number of occurrence of matches between a specific pressure amplitude ($\Delta P$) and a specific latency ($\Delta T$) related to successive measurements of single pressure waves over said time sequence.

42. A method according to claim 40, wherein a matrix is created based on determining numbers of single pressure waves with pre-selected values related to rise time coefficient ($\Delta P/\Delta T$) wherein one axis of the second matrix is related to an array of pre-selected values of rise time coefficient ($\Delta P/\Delta T$), and wherein each cell in said second matrix there is indicated occurrence of pre-selected rise time coefficients ($\Delta P/\Delta T$) related to successive measurements of single pressure waves over said time sequence.

43. A method according to claim 40, wherein the single pressure wave parameters are categorized into groups, said groups reflecting ranges of said single wave parameter values.

44. A method according to claim 40 , wherein reiterated updating of said matrix is made during said time sequence and during ongoing measurements taken within a measurement period.

45. A method according to claim 44, wherein said reiterated updating occurs in a time range of every 5-15 seconds.

46. (Previously Presented) A method according to anyone of claim 40, wherein said matrixes are computed for each consecutive time sequence in a series of repeated time sequences.

47. A method according to claims 40, wherein the occurrence of matches in said matrix is indicated through actual number or standardisation based number of matches during the specific measurement period.

48. A method according to claim 40, wherein the occurrence of matches is indicated through percentage of matches during the specific measurement period.

49. A method according to claim 40, wherein said standardisation of said numbers or percentages of occurrence of matches is a function of the length of the specific measurement period.

50. A method according to claim 47, wherein said standardisation is related to wavelength of a single pressure wave (heart rate).

51. A method according to claim 1, comprising the further step of computing balanced position for a number of occurrences of said single pressure wave amplitude ($\Delta P$) and latency ($\Delta T$) values in said first matrix.

52. A method according to claim 51, wherein balanced position of said first matrix of numbers of amplitude ($\Delta P$) and latency ($\Delta T$) combinations relates to mean frequency distribution of amplitude ($\Delta P$) and latency ($\Delta T$) combinations during said time sequence.

53. A method according to claim 1, wherein balanced position is computed for number of occurrences of said single pressure wave rise time coefficient ($\Delta P/\Delta T$) values in said second matrix.

54. A method according to claim 53, wherein said balanced position of said second matrix numbers of rise time coefficient ($\Delta P/\Delta T$) relates to mean frequency distribution of rise time coefficients ($\Delta P/\Delta T$) during said selected time sequence.

55. A method according to claim 51, wherein reiterated computation of said matrix balanced position within said time sequence is made during ongoing measurements taken over a measurement period.

56. A method according to claim 51, wherein a new matrix balanced position is computed for each time sequence in a consecutive series of said time sequences during ongoing measurements taken over a measurement period.

57. A method according to claim 55, wherein said reiterated updating is made in a time range of every 5-15 seconds.

58. A method according to claim 51, wherein balanced position of numbers of occurrences in said first or second matrix is presented as numerical values or as weighted values.

59. A method for analyzing pressure signals derivable from pressure measurements on or in a body of a human being or animal, comprising the steps of:

sampling said signals at specific intervals, and
converting thus sampled pressure signals into pressure-related digital data with a time reference,

wherein for selectable time sequences the method comprises the further steps of:

a) identifying from said digital data single wave parameters,
b) determining from said digital data time sequence parameters,
c) applying to said identified single wave parameters and determined time sequence parameters criteria for thresholds and ranges of said single wave parameters and time sequence parameters,
d) using said criteria for thresholds and ranges to provide for identification of single pressure waves related to cardiac beat-induced pressure waves and pressure waves caused by artifact-induced pressure waves or a combination thereof.

60. A method according to claim 59, wherein each of said selectable time sequences is a selected time duration of said pressure-related digital data with a time reference.

61. A method according to claim 60, wherein said selected duration lies in the range 5-15 seconds.

62. A method according to claim 59, wherein the method is applied to each of said selectable time sequences in a continuous series of said time sequences during a recording.

**63.** A method according to claim 59, wherein said identifying step a) includes identification of peaks and valleys in said sampled signal.

**64.** A method according to claim 63, wherein all minimum and maximum values are identified and represented with an amplitude value and a location value or time stamp.

**65.** A method according to claim 59, wherein said identifying step a) includes identification of included pair combinations of peaks and valleys in said sampled signal.

**66.** A method according to claim 59, wherein said identifying step a) includes identification of included pair combinations of valleys and peaks in said signal, corresponding to included pair combinations of diastolic minimum pressure ($P_{min}$) and systolic maximum pressure ($P_{max}$), characterizing single pressure waves created by the cardiac beat-induced pressure waves.

**67.** A method according to claim 59, wherein said identifying step a) includes identification of at least one of the single pressure wave parameters during said selected time sequences, said parameters selected from the group of:

- starting diastolic minimum pressure defining the start of the single pressure wave ($P_{min}$),
- ending diastolic minimum pressure defining the end of the single pressure wave ($P_{min}$),
- systolic maximum pressure of the single pressure wave ($P_{max}$),
- amplitude of the single pressure wave ($\Delta P$),
- latency of the single pressure wave ($\Delta T$),
- rise time coefficient of the single pressure wave ($\Delta P/\Delta T$),
- wavelength of the single pressure wave.

**68.** A method according to claim 67, wherein the ending diastolic minimum pressure ($P_{min}$) defines an end of a first single pressure wave which is same as starting diastolic minimum pressure ($P_{min}$) defining the start of the subsequent second single pressure wave.

**69.** A method according to claim 67, wherein the ending diastolic minimum pressure ($P_{min}$) defines an end of a first single pressure wave which is not the same as starting diastolic minimum pressure ($P_{min}$) defining the start of the subsequent second single pressure wave.

**70.** A method according to claim 67, wherein said amplitude of the single pressure wave ($\Delta P$) equals the pressure difference when pressures increase from starting diastolic minimum pressure ($P_{min}$) to systolic maximum pressure ($P_{max}$).

**71.** A method according to claim 67, wherein latency of the single pressure wave ($\Delta T$) equals the time interval of the single wave when the pressures change from starting diastolic minimum pressure ($P_{min}$) to systolic maximum pressure ($P_{max}$).

**72.** A method according to claim 67, wherein rise time coefficient ($\Delta P/\Delta T$) of the single pressure wave relates to amplitude of the single pressure wave ($\Delta P$) divided by latency ($\Delta T$) of the single pressure wave.

**73.** A method according to claim 67, wherein wavelength of each individual of said single pressure waves relates to the duration of the single pulse pressure wave between the diastolic minimum pressure ($P_{min}$) representing the start of the wave and the diastolic minimum pressure ($P_{min}$) representing the end of the wave.

**74.** A method according to claim 59, wherein said determining step b) includes determining at least one of the time sequence parameters during said individual time sequence, said parameters selected from the group of:

- number of single waves ($N_{SW}$),
- single pressure wave derived heart rate,
- absolute mean pressure,
- standard deviation for mean pressure of mean pressure for the individual single waves,
- standard deviation for diastolic minimum ($P_{min}$),
- standard deviation for systolic maximum ($P_{max}$),
- standard deviation for amplitude ($\Delta P$) of all individual single pressure waves,

34

- standard deviation for latency (ΔT) of all individual single pressure waves,
- standard deviation for rise time coefficient (ΔP/ΔT of all individual single pressure waves,
- balanced position of amplitude (ΔP)/latency (ΔT) combinations,
- balanced position of rise time coefficients (ΔP/ΔT).

**75.** A method according to claim 74, wherein said single pressure wave derived heart rate is computed as the number of single pressure waves divided with the total duration of wavelengths ($P_{min}$ to $P_{min}$) of single pressure waves within said time sequence.

**76.** A method according to claim 74, wherein said single pressure wave derived heart rate is computed as number of single pressure waves divided by the duration of said time sequence in which said single pressure waves occur.

**77.** A method according to claim 74, wherein said absolute mean pressure is computed as the sum of absolute mean pressure (entire wavelength from $P_{min}$ to $P_{min}$) for all individual single waves during said time sequence, divided by the number of single waves within said time sequence.

**78.** A method according to claim 74, wherein said balanced position of amplitude (ΔP)/latency (ΔT) combinations refers to the mean frequency distribution of single wave amplitude (ΔP)/latency (ΔT) combinations during said time sequences.

**79.** A method according to claim 74, wherein said balanced position of amplitude (ΔP)/latency (ΔT) combinations is computed in a first matrix of number of occurrences of amplitude (ΔP) and latency (ΔT) values for all individual single pressure waves during said time sequence.

**80.** A method according to claim 74, wherein said balanced position of rise time coefficients (ΔP/ΔT) refers to the mean frequency distribution of single wave rise time coefficients (ΔP/ΔT) during said time sequences.

**81.** A method according to claim 74, wherein said balanced position of rise time coefficients (ΔP/ΔT) is computed in a second matrix of number of occurrences of rise time coefficient (ΔP/ΔT) values for all individual single pressure waves during said time sequence.

**82.** A method according to claim 59, wherein said step c) includes use of criteria for thresholds and ranges of said single pressure wave parameters of said single pressure waves during said time sequences, said parameters selected from the group of:

- diastolic minimum pressure defining the start of the single pressure wave ($P_{min}$),
- ending diastolic minimum pressure defining the end of the single pressure wave ($P_{min}$),
- systolic maximum pressure of the single pressure wave ($P_{max}$),
- amplitude (ΔP) of the single pressure wave,
- latency (ΔT) of the single pressure wave,
- rise time coefficient (ΔP/ΔT) of the single pressure wave,
- wavelength of the single pressure wave.

**83.** A method according to claim 82, wherein said criteria for thresholds and ranges of said single pressure wave parameters determines accepting or rejecting said single pressure waves for further analysis.

**84.** A method according to claim 82, wherein said criteria for thresholds and ranges of said single pressure wave parameters exclude minimum-maximum pressure ($P_{min}$/ $P_{max}$) pairs with said single pressure wave parameters outside selectable thresholds and ranges.

**85.** A method according to claim 59, wherein said step c) includes use of criteria for thresholds and ranges of said time sequence parameters during said time sequence windows, said parameters selected from the group of:

- number of single waves ($N_{SW}$),
- single pressure wave derived heart rate,
- absolute mean pressure,
- standard deviation for mean pressure of mean pressure for the individual single waves,
- standard deviation for diastolic minimum ($P_{min}$),

- standard deviation for systolic maximum ($P_{max}$),
- standard deviation for amplitude ($\Delta P$) of all individual single pressure waves,
- standard deviation for latency ($\Delta T$) of all individual single pressure waves,
- standard deviation for rise time coefficient ($\Delta P/\Delta T$) of all individual single pressure waves,
- balanced position of amplitude ($\Delta P$)/latency ($\Delta T$) combinations,
- balanced position of rise time coefficients ($\Delta P/\Delta T$).

86. A method according to claim 85, wherein said criteria for thresholds and ranges of said time sequence parameters of said single pressure waves during said time sequences determines accepting or rejecting said time sequences for further analysis.

87. A method according to claim 59, wherein said identifying step a), determining step b) and step c) enables optimal identification of single pressure waves related to cardiac beat-induced pressure waves and identification of pressure waves related to artifacts or a combination of artifacts and cardiac beat-induced pressure waves.

88. A method according to claim 59, wherein said continuous pressure-related signals relate to single pressure waves created by physiological cardiac beat-induced pressure waves.

89. A method according to claim 59, wherein said identifying step a) and determining step b) further include selecting single pressure waves which occur between two consecutive ones of said time sequences and placing such waves in one or the other of said two consecutive individual time sequences according to selected criteria.

90. A method according to claim 89, wherein said selected criteria define that a first one of said single pressure waves within said individual time sequence windows has its ending diastolic minimum pressure value ($P_{min}$) within said individual time sequence.

91. A method according to claim 89, wherein said selected criteria define that a last of said single pressure waves within said individual time sequence must have both its starting ($P_{min}$) and ending ($P_{min}$) diastolic minimum pressure values within said individual time sequence window.

92. A method for analyzing pressure signals derivable from pressure measurements on or in a body of a human being or animal, comprising the steps of sampling said signals at specific intervals, and converting thus sampled pressure signals into pressure-related digital data with a time reference,
wherein for selectable time sequences the method comprises the further steps of:

a) identifying from said digital data single pressure waves related to cardiac beat-induced pressure waves,
b) computing time sequence parameters of said single pressure waves during individual of said time sequences, and
c) establishing an analysis output selected from one or more of said time sequence parameters of said single pressure waves during individual of said time sequences:

c1) balanced position of amplitude ($\Delta P$)/latency ($\Delta T$) combinations,
c2) balanced position of rise time coefficients ($\Delta P/\Delta T$)
c3) absolute mean pressure for said single pressure waves of said time sequence.

93. A method according to claim 92, wherein each of said selectable time sequences is a selected time duration of said pressure-related digital data with a time reference.

94. A method according to claim 93, wherein said selected time duration lies in the range 5 -15 seconds.

95. A method according to claim 92, wherein the method is applied to each of said selectable time sequences in a continuous series of said time sequences during a recording.

96. A method according to claim 92, wherein said identifying step a) includes identification of peaks and valleys in said sampled signal.

97. A method according to claim 96, wherein all minimum and maximum values are identified and represented with an amplitude value and a location value or time stamp.

**98.** A method according to claim 92, wherein said identifying step a) includes identification of included pair combinations of peaks and valleys in said signal.

**99.** A method according to claim 92, wherein said identifying step a) includes identification of included pair combinations of valleys and peaks in said signal, corresponding to included pair combinations of diastolic minimum pressure ($P_{min}$) and systolic maximum pressure ($P_{max}$), characterizing single pressure waves created by the cardiac beat-induced pressure waves.

**100.** A method according to claim 92, wherein said identifying step a) excludes for further analysis pressure waves during said time sequences with single pressure wave parameters outside selected criteria for thresholds and ranges of said parameters, said parameters selected from the group of:

- starting diastolic minimum pressure defining the start of the single pressure wave ($P_{min}$),
- ending diastolic minimum pressure defining the end of the single pressure wave ($P_{min}$),
- systolic maximum pressure of the single pressure wave ($P_{max,}$),
- amplitude ($\Delta P$) of the single pressure wave,
- latency ($\Delta T$) of the single pressure wave,
- rise time coefficient ($\Delta P/\Delta T$) of the single pressure wave,
- wave duration of the single pressure wave, and
- absolute mean pressure of said single pressure wave.

**101.** A method according to claim 92, wherein said identifying step a) includes for further analysis single pressure waves having single pressure wave parameters within selected criteria for thresholds and ranges of said single pressure wave parameters.

**102.** A method according to claim 92, wherein said identifying step a) excludes for further analysis time sequences with time sequence parameters outside selected criteria for thresholds and ranges of said parameters, said parameters selected from the group of:

- number of single waves ($N_{SW}$),
- single pressure wave derived heart rate,
- absolute mean pressure,
- standard deviation for mean pressure of mean pressure for the individual single waves,
- standard deviation for diastolic minimum ($P_{min}$),
- standard deviation for systolic maximum ($P_{max}$),
- standard deviation for amplitude ($\Delta P$) of all individual single pressure waves,
- standard deviation for latency ($\Delta T$) of all individual single pressure waves,
- standard deviation for rise time coefficient ($\Delta P/\Delta T$) of all individual single pressure waves,
- balanced position of amplitude ($\Delta P$)/latency ($\Delta T$) combinations,
- balanced position of rise time coefficients ($\Delta P/\Delta T$)

**103.** A method according to claim 92, wherein said identifying step a) includes for further analysis time sequences having time sequence parameters within selected criteria for thresholds and ranges of said time sequence parameters.

**104.** A method according to claim 92, wherein said identifying step a) is applied to each consecutive time sequence in a continuous series of time sequences of a signal.

**105.** A method according to claim 92, wherein said identifying step a) further includes selecting single pressure waves which occur between two consecutive of said time sequences and placing such waves in one or the other of said two consecutive individual time sequences according to selected criteria.

**106.** A method according to claim 105, wherein said selected criteria define that a first of said single pressure waves within said individual time sequence must have its ending diastolic minimum pressure value ($P_{min}$) within said individual time sequence.

**107.** A method according to claim 105, wherein said selected criteria define that a last of said single pressure waves within said individual time sequence must have both its starting ($P_{min}$) and ending ($P_{min}$) diastolic minimum pressure values within said individual time.

**108.** A method according to claim 92, wherein said computing step b) for accepted time sequences further includes determining said time sequence parameters, said parameters selected from the group of:

c1) balanced position of amplitude (ΔP)/latency (ΔT) combinations,
c2) balanced position of rise time coefficients (ΔP/ΔT),
c3) absolute mean pressure for said single pressure waves of said time sequence.

**109.** A method according to claim 92, wherein said establishing step c) includes determining balanced position of amplitude (ΔP)/latency (ΔT) combinations, said determining comprising the steps of creating a first matrix based on determining number of single pressure waves with pre-selected values related to amplitude (ΔP) and latency (ΔT), one axis of said first matrix being related to an array of pre-selected values of pressure amplitude (ΔP) and the other axis of said first matrix being related to an array of pre-selected values of latencies (ΔT), and indicating for each matrix cell at respective intersections in said first matrix a number of occurrences of matches between a specific pressure amplitude (ΔP) and a specific latency (ΔT) related to successive measurements of single pressure waves over said individual time sequences.

**110.** A method according to claim 109, wherein the single pressure wave parameters of amplitude (ΔP) and latency (ΔT) are categorized into groups, said groups reflecting ranges of said single wave parameter values.

**111.** A method according to claim 109, wherein the occurrence of matches in said first matrix is indicated through actual number of matches during individual of said time sequence windows.

**112.** A method according to claim 109, comprising the further step of computing balanced position for a number of occurrences of said single pressure wave parameters of amplitude (ΔP) and latency (ΔT) values during individual of said time sequences in said first matrix.

**113.** A method according to claim 112, wherein said balanced position of said first matrix of numbers of amplitude (ΔP) and latency (ΔT) combinations corresponds to mean frequency distribution of the different occurrences of amplitude (ΔP) and latency (ΔT) during said individual time sequences.

**114.** A method according to claim 92, wherein said establishing step c) includes determining balanced position of rise time coefficients (ΔP/ΔT), said determining comprising the steps of creating a second matrix based on determining number of single pressure waves with pre-selected values related to rise time coefficient (ΔP/ΔT), the axis in said second matrix being related to an array of pre-selected values of rise time coefficient (ΔP/ΔT), and wherein for each matrix cell in said second matrix indicating a number of occurrences of pre-selected rise time coefficients (ΔP/ΔT) related to successive measurements of single pressure waves during said individual time sequences.

**115.** A method according to claim 114, wherein the single pressure wave parameter rise time coefficient (ΔP/ΔT) is categorized into groups, said groups reflecting ranges of said single wave (ΔP/ΔT) parameter values.

**116.** A method according to claim 114, comprising the further step of computing balanced position for a number of occurrences of said single pressure wave parameter rise time coefficient (ΔP/ΔT) in said second matrix, to yield an analysis output.

**117.** A method according to claim 116, wherein said balanced position of said second matrix of numbers of rise time coefficient (ΔP/ΔT) combinations corresponds to the mean frequency distribution of rise time coefficient (ΔP/ΔT) of said time sequence.

**118.** A method according to claim 92, wherein said establishing step c) yields analysis output related to the absolute mean pressure for said single pressure waves of said time sequence, corresponding to the sum of mean pressure values for all individual single pressure waves during said time sequence divided by number of said individual single pressure waves during said individual time sequence.

**119.** A method according to claim 118, wherein absolute mean pressure for an individual of said single pressure waves is the sum of sample values during the time of a wave duration, i.e. from starting diastolic minimum pressure ($P_{min}$) to ending diastolic minimum pressure ($P_{min}$) divided by number of samples.

**120.** A method according to claim 92, wherein said establishing step c) yields output of analysis of parameters c1) - c3) during each individual of said time sequence windows in a continuous series of said time sequence windows of said pressure-related signal.

121. A method according to claim 92, wherein the duration of each selectable time sequence window lies in a time range of 3 -15 seconds.

122. A method according to claim 92, wherein establishing step c) yields output of analysis of one or more of said parameters c1)- c3 ), said analysis output being presented as numerical values on a display for each of said time sequences during ongoing sampling of said pressure-related signals.

123. A method according to claim 92, wherein establishing step c) yields output of analysis of one or more of parameters c1) - c3), said analysis output being presented as histogram distribution of values of said parameters c1) - c3) for a selectable number of time sequence windows of said pressure-related signal.

124. A method according to claim 92, wherein establishing step c) yields output of analysis of one or more of parameters c1) - c3), said analysis output being presented as a quantitative matrix for a selectable number of time sequences of said pressure-related signal.

125. A method according to claim 124, wherein said quantitative matrix is created based on determining numbers of one of said parameters c1) - c3) with selected parameter values, wherein one axis of the quantitative matrix is related to an array of selected parameter values, wherein the other axis is related to an array of selected numbers of consecutive included time sequences, and wherein indicating for each matrix cell at respective intersections in said quantitative matrix a number of occurrence of matches between a specific parameter value and a specific number of time sequences.

126. A method according to claim 125, wherein said parameter values are categorized into groups, said groups reflecting ranges of said parameter values.

127. A method for analysing pressure-signals derivable from pressure measurements on or in a body of a human being or animal, comprising the steps of sampling said signals at specific intervals and converting the pressure-signals into pressure related digital data with a time reference,
wherein for selectable time sequences the method further comprises the steps of

a) identifying from said digital data single pressure waves related to cardiac beat-induced pressure waves,
b) computing time sequence parameters of said single pressure waves during individual of said time sequences,
c) establishing an analysis output selected from one or more of said time sequence parameters of said single pressure waves during individual of said time sequences:

c1) - absolute mean pressure for each identified single pressure wave (wavelength $P_{min}$ - $P_{min}$) within said time sequence,
c2) - mean of mean pressure for all identified single pressure waves (wavelength $P_{min}$- $P_{min}$) within said time sequence,
c3) - standard deviation of absolute mean pressure for all identified single pressure waves (wavelength $P_{min}$ - $P_{min}$) within said time sequence,
c4) - numbers of single pressure waves during said time sequence,
c5) - single pressure wave derived heart rate during said time sequence,
c6) - relative pressure amplitude ($\Delta P$) value for each identified single pressure wave (wavelength $P_{min}$ - $P_{min}$) within said time sequence,
c7) - standard deviation of relative pressure amplitude ($\Delta P$) values for all identified single pressure waves (wavelength $P_{min}$ - $P_{min}$) within said time sequence,
c8) - relative latency ($\Delta T$) value for each identified single pressure wave (wavelength $P_{min}$ - $P_{min}$) within said time sequence,
c9) - standard deviation of relative latency ($\Delta T$) values for all identified single pressure waves (wavelength $P_{min}$ - $P_{min}$) within said time sequence,
c10) - rise time ($\Delta P/\Delta T$) coefficient for each identified single pressure wave (wavelength $P_{min}$ - $P_{min}$) within said time sequence,
c11) - standard deviation of rise time ($\Delta P/\Delta T$) coefficients for all identified single pressure waves (wavelength $P_{min}$- $P_{min}$) within said time sequence,

c12) - balanced position within a first matrix for combinations of single pressure wave amplitude ($\Delta P$) and latency ($\Delta T$) values within said time sequence,

c13) - balanced position within a second matrix for combinations of single pressure wave rise-time ($\Delta P/\Delta T$) coefficient values within said time sequence,

d) establishing a deliverable first control signal related to an analysis output in step c) for a selectable number of said time sequence windows, said first control signal being determined according to one or more selectable criteria for said analysis output, and

e) modifying said deliverable first control signal into a regulator deliverable second control signal said second control signal corresponding to said first deliverable control signal, and

f) to provide a performance modifying signal.

**128.** A method according to claim 127, wherein each of said selectable time sequences is a selected time duration of said pressure-related digital data with a time reference.

**129.** A method according to claim 127, wherein said selected time duration lies in the range 3 -15 seconds.

**130.** A method according to anyone of claim 127, wherein the method is applied to each of said selectable time sequences in a continuous series of said time sequences during a recording.

**131.** A method according to claim 127, wherein said identifying step a) includes identification of peaks and valleys in said sampled signal.

**132.** A method according to claim 127, wherein all minimum and maximum values are identified and represented with an amplitude value and a location value or time stamp.

**133.** A method according to claim 127, wherein said identifying step a) includes identification of included pair combinations of peaks and valleys in said signal.

**134.** A method according to claim 128, wherein said identifying step a) includes identification of included pair combinations of valleys and peaks in said signal, corresponding to included pair combinations of diastolic minimum pressure ($P_{min}$) and systolic maximum pressure ($P_{max}$), characterizing single pressure waves created by the cardiac beat-induced pressure waves.

**135.** A method according to claim 127, wherein said identifying step a) excludes for further analysis pressure waves during said time sequences with single pressure wave parameters outside selected criteria for thresholds and ranges of said parameters, said parameters selected from the group of:

- starting diastolic minimum pressure defining the start of the single pressure wave ($P_{min}$),
- ending diastolic minimum pressure defining the end of the single pressure wave ($P_{min}$),
- systolic maximum pressure of the single pressure wave ($P_{max}$),
- amplitude of the single pressure wave ($\Delta P$),
- latency of the single pressure wave ($\Delta T$),
- rise time coefficient of the single pressure wave ($\Delta P/\Delta T$),
- wave duration of the single pressure wave, and
- absolute mean pressure of said single pressure wave.

**136.** A method according to claim 127, wherein said identifying step a) includes for further analysis single pressure waves having single pressure wave parameters within selected criteria for thresholds and ranges of said single pressure wave parameters.

**137.** A method according to claim 127, wherein said identifying step a) excludes for further analysis time sequences with time sequence parameters outside selected criteria for thresholds and ranges of said parameters, said parameters selected from the group of:

- numbers of single waves ($N_{SW}$),
- single pressure wave derived heart rate,
- absolute mean pressure,

- standard deviation for mean pressure of mean pressure for the individual single waves,
- standard deviation for diastolic minimum ($P_{min}$),
- standard deviation for systolic maximum ($P_{max}$),
- standard deviation for amplitude ($\Delta P$) of all individual single pressure waves,
- standard deviation for latency ($\Delta T$) of all individual single pressure waves,
- standard deviation for rise time coefficient ($\Delta P/\Delta T$) of all individual single pressure waves,
- balanced position of amplitude ($\Delta P$)/latency ($\Delta T$) combinations,
- balanced position of rise time coefficients ($\Delta P/\Delta T$).

**138.** A method according to claim 127, wherein said identifying step a) includes for further analysis time sequences having time sequence parameters within selected criteria for thresholds and ranges of said time sequence parameters.

**139.** A method according to 127, wherein said identifying step a) is applied to each consecutive time sequence in a continuous series of time sequences of a signal.

**140.** A method according to claim 127, wherein said identifying step a) further includes selecting single pressure waves which occur between two consecutive ones of said time sequences and placing such waves in one or the other of said two consecutive individual time sequences according to selected criteria.

**141.** A method according to claim 140, wherein said selected criteria define that a first one of said single pressure waves within said individual time sequence has its ending diastolic minimum pressure value ($P_{min}$) within said individual time sequence.

**142.** A method according to claim 141, wherein said selected criteria define that a last one of said single pressure waves within said individual time sequence has both its starting ($P_{min}$) and ending ($P_{min}$) diastolic minimum pressure values within said individual time sequence window.

**143.** A method according to claim 127, wherein said computing step b) for accepted time sequences further includes determining said time sequence parameters, said parameters selected from the group of:

- numbers of single waves ($N_{SW}$),
- single pressure wave derived heart rate,
- absolute mean pressure,
- standard deviation for mean pressure of mean pressure for the individual single waves,
- standard deviation for diastolic minimum ($P_{min}$),
- standard deviation for systolic maximum ($P_{max}$),
- standard deviation for amplitude ($\Delta P$) of all individual single pressure waves,
- standard deviation for latency ($\Delta T$) of all individual single pressure waves,
- standard deviation for rise time coefficient ($\Delta P/\Delta T$) of all individual single pressure waves,
- balanced position of amplitude ($\Delta P$)/latency ($\Delta T$) combinations,
- balanced position of rise time coefficients ($\Delta P/\Delta T$).

**144.** A method according to claim 127, wherein said establishing step c) includes determining balanced position of amplitude ($\Delta P$)/latency ($\Delta T$) combinations, said determining comprising the steps of creating a first matrix based on determining number of single pressure waves with pre-selected values related to amplitude ($\Delta P$) and latency ($\Delta T$), one axis of said first matrix being related to an array of pre-selected values of pressure amplitude ($\Delta P$) and the other axis of said first matrix being related to an array of pre-selected values of latencies ($\Delta T$), and indicating for each matrix cell at respective intersections in said first matrix a number of occurrences of matches between a specific pressure amplitude ($\Delta P$) and a specific latency ($\Delta T$) related to successive measurements of single pressure waves over said individual time sequences.

**145.** A method according to claim 144, wherein the single pressure wave parameters of amplitude ($\Delta P$) and latency ($\Delta T$) are categorized into groups, said groups reflecting ranges of said single wave parameter values.

**146.** A method according to claim 144, wherein the occurrence of matches in said first matrix is indicated through actual number of matches during individual of said time sequence windows.

**147.** A method according to claim 144, comprising the further step of computing balanced position for a number of occurrences of said single pressure wave parameters of amplitude ($\Delta P$) and latency ($\Delta T$) values during individual of said time sequences in said first matrix.

**148.** A method according to claim 147, wherein said balanced position of said first matrix of numbers of amplitude ($\Delta P$) and latency ($\Delta T$) combinations corresponds to mean frequency distribution of the different occurrences of amplitude ($\Delta P$) and latency ($\Delta T$) during said individual time sequences.

**149.** A method according to claim 127, wherein said establishing step c) includes determining balanced position of rise time coefficients ($\Delta P/\Delta T$), said determining comprising the steps of creating a second matrix based on determining number of single pressure waves with pre-selected values related to rise time coefficient ($\Delta P/\Delta T$), the axis in said second matrix being related to an array of pre-selected values of rise time coefficient ($\Delta P/\Delta T$), and wherein for each matrix cell in said second matrix indicating a number of occurrences of pre-selected rise time coefficients ($\Delta P/\Delta T$) related to successive measurements of single pressure waves during said individual time sequences.

**150.** A method according to claim 149, wherein the single pressure wave parameter rise time coefficient ($\Delta P/\Delta T$) is categorized into groups, said groups reflecting ranges of said single wave ($\Delta P/\Delta T$) parameter values.

**151.** A method according to claim 149, comprising the further step of computing balanced position for a number of occurrences of said single pressure wave parameter rise time coefficient ($\Delta P/\Delta T$) in said second matrix, to yield an analysis output.

**152.** A method according to claim 151, wherein said balanced position of said second matrix of numbers of rise time coefficient ($\Delta P/\Delta T$) combinations corresponds to the mean frequency distribution of rise time coefficient ($\Delta P/\Delta T$) of said time sequence.

**153.** A method according to claim 127, wherein said establishing step c) yields analysis output related to the parameter c2) mean of mean pressure for said single pressure waves of said time sequence, corresponding to the sum of mean pressure values for all individual single pressure waves during said time sequence divided by number of said individual single pressure waves during said individual time sequence.

**154.** A method according to claim 127, wherein the parameter c1) absolute mean pressure for an individual of said single pressure waves is the sum of sample values during the time of a wave duration, i.e. from starting diastolic minimum pressure ($P_{min}$) to ending diastolic minimum pressure ($P_{min}$) divided by number of samples.

**155.** A method according to claim 137, wherein said establishing step c) yields output of analysis of one or more of parameters c1) - c13) during each individual of said time sequence windows in a continuous series of said time sequence windows of said pressure-related signal.

**156.** A method according to claim 127, wherein the duration of each selectable time sequence window lies in a time range of 3 -15 seconds.

**157.** A method according to claim 127, wherein said establishing step c) relates to obtaining an analysis output based on said time sequence parameters for a selectable number of said individual time sequence windows.

**158.** A method according to claim 127, wherein said analysis output is a mean value of balanced position of amplitude ($\triangle P$)/latency ($\triangle T$) combinations for a selectable number of said individual time sequence windows.

**159.** A method according to claim 127, wherein said analysis output is a mean value of balanced position of rise-time ($\Delta P/\triangle T$) coefficient values for a selectable number of said individual time sequence windows.

**160.** A method according to claim 127, wherein said analysis is performed by a processing unit, said processing unit delivering a first control signal to a regulator, said first control signal being determined according to selectable criteria for output of said analysis.

**161.** A method according to claim 127, wherein output of said analysis of single pressure wave related digital data for a given time sequence yields modification of said deliverable first control signal.

**162.** A method according to claim 127, wherein features of said first control signal are selectable when output of said single pressure wave analysis derivable from a subsequent pressure monitoring meet specific criteria.

**163.** A method according to claim 127, wherein said selected criteria of single pressure wave related parameters relate to criteria for optimum single pressure wave detection.

**164.** A method according to claim 127, wherein said selectable first control signal is determined during each individual time sequence in a continuous series of time sequences, according to output of said single pressure wave analysis for each time sequence in said continuous series of time sequences.

**165.** A method according to claim 127, wherein said first control signal is converted within a regulator to a deliverable second control signal, said selectable second control signal corresponding to said selectable first control signal.

**166.** A method according to claim 165, wherein said regulator deliverable second control signal provides a performance modifying signal, said performance modifying signal causing causes modifications in the performance of a sensor-regulating device.

**167.** A method according to claim 166, wherein said sensor-regulating device is adjustable according to a regulator deliverable second control signal.

**168.** A method according to claim 165, wherein said sensor-regulating device modifies the mode by which the sensor is able to sample signals indicative of pressure.

**169.** A method according to claim 127, wherein there is feedback between a processing unit performing single pressure wave analysis controlling a deliverable first control signal to a regulator, and a regulator controlling a deliverable second control signal to a sensor-regulating device, said feedback signal being reiterable at selected intervals during an ongoing pressure measurement.

**170.** A method according to claim 169, wherein output of said single pressure wave analysis for a given time sequence is related to said second deliverable control signal.

**171.** A method according to claims 170, wherein output of said single pressure wave analysis is determined for each individual time sequence during a series of continuous time sequences, wherein said second control signal is modified to another level between each of said individual time sequences, and the level of said second control signal is related to said analysis output for each of said time sequences.

**172.** A method according to claims 170, wherein said first control signal is determined according to said second control signal, or said second control signal is determined according to said first control signal.

**173.** A method according to claim 127, wherein the deliverable first and second control signals relate to output of said single pressure wave analysis indicative of optimum single pressure wave detection, said first and second control signals being used during the subsequent pressure monitoring.

**174.** A method according to claim 127, wherein said human or animal body pressure is one or more of intracranial pressure, arterial blood pressure, cerebrospinal fluid pressure, cerebral perfusion pressure, ocular pressure, gastrointestinal pressure, urinary tract pressure, or any type of soft tissue pressure.

**175.** A system for analysing pressure-signals derivable from pressure measurements on or in a body of a human being or animal, said system comprising:

a) control means which on basis of said pressure signals receivable from a pressure sensor via pressure transducer means is configured to control performance of a pressure sensor regulating device to optimize single pressure wave detection,
b) a processing unit having means for analyzing said pressure signals, said processing unit including sampling means for sampling said pressure signals at specific intervals,
c) converter means for converting the sampled pressure signals into pressure related digital data with a time reference,
d) identifying means operative during selectable time sequences to identify from said digital data single pressure waves related to one of: cardiac beat-induced pressure waves, artifacts, and a combination of cardiac beat-induced waves and artifacts,

e) analyzing means for analysis of said digital data single pressure waves during said selectable time sequences,

f) output means configured to output to a regulator device one or more first control signals derivable from one or more time sequence parameters related to a selectable number of time sequences elected from the group of:

f1) absolute mean pressure for each identified single pressure wave (wavelength $P_{min}$- $P_{min}$) within said time sequence,

f2) mean of mean pressure for all identified single pressure waves (wavelength $P_{min}$- $P_{min}$) within said time sequence,

f3) standard deviation of absolute mean pressure for all identified single pressure waves (wavelength $P_{min}$ - $P_{min}$) within said time sequence,

f4) numbers of single pressure waves during said time sequence,

f5) single pressure wave derived heart rate during said time sequence,

f6) relative pressure amplitude ($\Delta P$) value for each identified single pressure wave (wavelength $P_{min}$- $P_{min}$) within said time sequence,

f7) standard deviation of relative pressure amplitude ($\Delta P$) values for all identified single pressure waves (wavelength $P_{min}$- $P_{min}$) within said time sequence,

f8) relative latency ($\Delta T$) value for each identified single pressure wave (wavelength $P_{min}$ - $P_{min}$) within said time sequence,

f9) standard deviation of relative latency ($\Delta T$) values for all identified single pressure waves (wavelength $P_{min}$- $P_{min}$) within said time sequence,

f10) rise time ($\Delta P/\Delta T$) coefficient for each identified single pressure wave (wavelength $P_{min}$- $P_{min}$) within said time sequence,

f11) standard deviation of rise time ($\Delta P/\Delta T$) coefficients for all identified single pressure waves (wavelength $P_{min}$- $P_{min}$) within said time sequence,

f12) balanced position within a first matrix for combinations of single pressure wave amplitude ($\Delta P$) and latency ($\Delta T$) values within said time sequence, and

f13) balanced position within a second matrix for combinations of single pressure wave rise-time ($\Delta P/\Delta T$) coefficient values within said time sequence, and

g) regulator means connectable to said processing unit for receiving at least one of said first control signals, said regulator means being capable of establishing a device performance modifying second control signal by means of at least one of said first control signals or establishing a combination effect obtained from using at least two of said first control signals, wherein said performance modifying second control signal deliverable from said regulator means being capable of controlling said sensor regulating device.

176. A system according to claim 175, wherein said sensor provides for sensing pressure signals related to arterial blood pressure.

177. A system according to claim 175, wherein said sensor provides for sensing pressure signals related to ocular bulb pressure.

178. A system according to claim 175, wherein said sensor provides for sensing pressure signals related to intracranial pressure.

179. A system according to claim 175, wherein said pressure-signals are arterial applanation pressure signals, being transformable into pressure signals indicative of arterial blood pressure signals.

180. A system according to claim 175, wherein said pressure signals are ocular applanation pressure signals, being transformable into pressure signals indicative of ocular bulb pressure signals.

181. A system according to claim 175, wherein said pressure-signals are fontanel applanation pressure signals, being transformable into pressure signals indicative of intracranial pressure signals.

182. A system according to claim 175, wherein said pressure signals are Doppler signals, being transformable into pressure signals.

**183.** A system according to claim 175, wherein said pressure signals are acoustic signals, being transformable into pressure signals.

**184.** A system according to claim 175, wherein said selectable time sequences are selected time durations of said pressure-related digital data with a time reference.

**185.** A system according to claim 184, wherein said selected time duration lies in the range 3 -15 seconds.

**186.** A system according to claim 175, wherein said analysis is performed by a processing unit, said processing unit delivering a first control signal to a regulator, said first control signal being determined according to selectable criteria for output of said analysis.

**187.** A system according to claim 175, wherein output of said analysis of single pressure wave related digital data for a given time sequence yields modification of said deliverable first control signal.

**188.** A system according to claim 175, wherein features of said first control signal are selectable when output of said single pressure wave analysis derivable from a subsequent pressure monitoring meet specific criteria.

**189.** A system according to claim 175, wherein said selected criteria of time sequence parameters relate to criteria for optimum single pressure wave detection.

**190.** A system according to claim 175, wherein said selectable first control signal is determined during each individual time sequence in a continuous series of time sequences, according to output of said single pressure wave analysis for each time sequence in said continuous series of time sequences.

**191.** A system according to claim 175, wherein said regulator means is configured to convert said first control signal into a deliverable second control signal, said selectable second control signal corresponding to said selectable first control signal.

**192.** A system according to claim 175, wherein said second control signal delivered by said regulator constitutes a performance modifying signal, said performance modifying signal being an input to a sensor-regulating device to cause modifications of performance thereof.

**193.** A system according to claim 175, wherein said sensor-regulating device is adjustable according to a regulator deliverable second control signal.

**194.** A system according to claim 175, wherein said sensor-regulating device is operable to modify a mode by which the sensor is able to sample signals indicative of pressure.

**195.** A system according to claim 175, wherein said processing unit is operable to perform single pressure wave analysis in order to control a first control signal which is deliverable to a regulator, said regulator operable to control a second control signal which is deliverable to a sensor-regulating device, and wherein a feedback signal is provided between said processing unit said regulator, said feedback signal being reiterable at selected intervals during an ongoing pressure measurement.

**196.** A system according to claim 175, wherein output of said single pressure wave analysis for a given time sequence is related to said second deliverable control signal.

**197.** A system according to claim 175, wherein determining means are provided to determine an output of said single pressure wave analysis for each individual time sequence during a series of continuous time sequences, and wherein modifying means are operative to modify said second control signal into another level between each of said individual time sequences, said level of said second control signal being related to said analysis output for each of said time sequences.

**198.** A system according to claim 175, wherein said first control signal is is a function of said second control signal, or said second control signal is a function of said first control signal.

**199.** A system according to claim 175, wherein the deliverable first and second control signals relate to output of said single pressure wave analysis which is indicative of optimum single pressure wave detection, said first and second control signals being for use during the subsequent pressure monitoring.

200. A system according to claim 175, wherein said human or animal body pressure is selected from one or more of: intracranial pressure, arterial blood pressure, cerebrospinal fluid pressure, cerebral perfusion pressure, ocular pressure, gastrointestinal pressure, urinary tract pressure, any type of soft tissue pressure.

Fig. 1a

Fig. 1b

EP 1 770 545 A2

Fig. 2a

Fig. 2b

*Fig. 3a*

*Fig. 3b*

Fig. 4a

Fig. 4b

Fig. 4c

Fig. 5a

Fig. 5b

Fig. 6a

Fig. 6b

Fig. 6c

Fig. 7a

Fig. 7b

Fig. 7c

EP 1 770 545 A2

*Fig. 8a*

Time sequence (5 sec) — 11

*Fig. 8b*

| dP/dT | 1.0 | 1.5 | 2.0 | 2.5 |
|-------|-----|-----|-----|-----|
| 0.10  |     |     |     |     |
| 0.11  |     |     | 3   |     |
| 0.12  |     |     |     | 1   |
| 0.13  |     |     | 2   |     |
| 0.14  |     |     |     | 1   |

*Fig. 8c*

**Balanced position** — 38

**0.12 seconds|2.4 mmHg**

*Fig. 8f*

*Fig. 8e*

*Fig. 8d*

EP 1 770 545 A2

Fig. 9a

Fig. 9b

Fig. 9c

Fig. 9d

Fig. 10c

Fig. 10a

Fig. 10d

Fig. 10b

Fig 11a

Fig 11b

Fig 11c

Fig 11d

Fig 12a

Control signal (51)

Regulator (52)

Control signal (50)

Sensor-regulating device (48)

Sensor (46)

Transducer (47)

Processing unit (49)

Fig 12b

Fig 12c

Control signal (53)

1st (11)  2nd  3rd  4th  5th (54)  6th (11)  7th

Time (seconds) (12)

EP 1 770 545 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 09843702 B

- WO 0200164 A